(19)

Europäisches
Patentamt
European
Patent Office
Office européen
des brevets

(11) **EP 4 450 514 A2**

(12) **EUROPEAN PATENT APPLICATION**

(43) Date of publication:
**23.10.2024 Bulletin 2024/43**

(21) Application number: **24190938.1**

(22) Date of filing: **14.10.2020**

(51) International Patent Classification (IPC):
**C07K 14/195** [(2006.01)]

(52) Cooperative Patent Classification (CPC):
**A61K 48/005; A61P 9/10; C07K 14/195; C07K 14/21; C12N 15/86;** A61K 38/00; C12N 2740/16043; C12N 2750/14143

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2019 US 201962914722 P**

(62) Document number(s) of the earlier application(s) in accordance with Art. 76 EPC:
**20877808.4 / 4 045 093**

(71) Applicant: **Duke University**
**Durham, NC 27705 (US)**

(72) Inventors:
• **WU, Tianyu**
**Durham, 27705 (US)**
• **BURSAC, Nenad**
**Durham, 27705 (US)**
• **NGUYEN, Hung**
**Durham, 27705 (US)**

(74) Representative: **Harris, Oliver John Richard**
**Novagraaf UK**
**Centrum**
**Norwich Research Park**
**Colney Lane**
**Norwich NR4 7UG (GB)**

Remarks:
•The complete document including Reference Table(s) and the Sequence Listing(s) can be downloaded from the EPO website
•This application was filed on 25-07-2024 as a divisional application to the application mentioned under INID code 62.

(54) **COMPOSITIONS COMPRISING NOVEL PROKARYOTIC SODIUM CHANNELS AND ASSOCIATED METHODS**

(57) Provided herein are compositions and methods for treating cardiac conditions and other diseases. In particular, the disclosure provides compositions and methods for the delivery of sodium channels. The compositions are particularly suitable in gene therapy applications and for cardiac tissue patch implantations.

EP 4 450 514 A2

**Fig. 1A**

**Description**

**CROSS-REFERENCES TO RELATED APPLICATIONS**

This application claims the benefit of and priority to U.S. Provisional Application No. 62/914,722 filed on October 14, 2019, which is hereby incorporated by reference in its entirety.

**STATEMENT REGARDING FEDERALLY FUNDED RESEARCH**

This invention was made with Government support under Grant Numbers R01 HL126524, R01 HL132389, and U01 HL134764 awarded by the National Institutes of Health. The Government has certain rights in the invention.

**REFERENCE TO A SEQUENCE LISTING SUBMITTED AS A TEXT FILE VIA EFS-WEB**

The official copy of the sequence listing is submitted electronically via EFS-Web as an ASCII formatted sequence listing with a file named 1212501_seqlist.txt, created on October 13, 2020, and having a size of 122 kb and is filed concurrently with the specification. The sequence listing contained in this ASCII formatted document is part of the specification and is herein incorporated by reference in its entirety.

**BACKGROUND**

Myocardial infarction, heart failure, and sudden cardiac death continue to have a devastating impact on public health, shortening lifespan and impairing the quality of life. In response to an acute myocardial injury that can obliterate hundreds of millions cardiomyocytes, the surviving heart responds with fibrotic scarring and often adversely remodels to functionally deteriorate or fail. Loss of cardiomyocytes and tissue fibrosis often cause lethal arrhythmias due to impaired cardiac contraction and electrical conduction. Therapies currently in use for restoring cardiac function each have limitations that influence efficacy, safety, or applicability. For example, adult-derived stem cell delivery has shown limited success, primarily because cells employed for therapy do not generate functional cardiomyocytes. While human pluripotent stem cell (hPSC)-derived cardiomyocytes (hPSC-CMs) address this issue and can be produced in unlimited numbers, their ultimate success will depend on their ability to approximate adult cardiomyocyte phenotype, survive, and functionally integrate *in vivo*. Specifically, immature hPSC-CMs may cause arrhythmias or lack any therapeutic effects, the latter being exacerbated by their low engraftment when injected in infarcted hearts. As an alternative, tissue engineering strategies have been developed. However, challenges with regard to maturation, thickness, functional integration, and invasiveness of delivery remain to hinder potential translation of this therapeutic strategy to clinical applications. Similarly, currently available strategies for ventricular rate control and/or sinus rhythm restoration by drug or ablation therapies are suboptimal. Antiarrhythmic drugs carry arrhythmogenic risks and non-cardiovascular toxicities while ablation therapies have high recurrence rates requiring repeated procedures and can lead to severe complications.

**BRIEF SUMMARY**

Provided herein is a nucleic acid comprising a nucleotide sequence encoding a sodium channel polypeptide comprising an amino acid sequence having at least 90% identity to the sequence of any one of SEQ ID NOs: 1-29, wherein the nucleotide sequence is operatively linked to a heterologous promoter. In some embodiments, the nucleotide sequence is selected from the group consisting of SEQ ID NOs: 30-58. Also provided herein is a nucleotide sequence encoding a sodium channel polypeptide comprising an amino acid sequence having at least 90% identity to the sequence of any one of SEQ ID NOs: 1-29, where the nucleotide sequence is not a naturally occurring sequence encoding the sodium channel polypeptide.

Also provided herein is a vector comprising a nucleic acid comprising a nucleotide sequence encoding a sodium channel polypeptide comprising an amino acid sequence having at least 90% identity to the sequence of any one of SEQ ID NOs: 1-29, wherein the nucleotide sequence is operatively linked to a heterologous promoter. In some embodiments, the vector may be a viral vector, such as a an adeno-associated viral (AAV) vector, a lentiviral vector, or a retroviral vector.

Also provided is a virus (e.g., an AAV, a lentivirus, or a retrovirus) comprising any of the nucleic acids or vectors described in this disclosure.

Also provided is a cell comprising any of the nucleic acids, vectors, or viruses described in this disclosure.

Provided herein is also a pharmaceutical composition comprising any of the nucleic acids, vectors, viruses, or cells described herein, and a pharmaceutically acceptable excipient.

Also provided is a tissue patch comprising a plurality of any of the cells described in this disclosure.

The disclosure further includes a method of treating a cardiac condition characterized by impaired action potential conduction in the heart, comprising administering to a subject in need thereof a therapeutically effective amount of any of the nucleic acids, vectors, viruses, cells, or pharmaceutical compositions described in this disclosure. In some embodiments, the cardiac condition may be one or more of cardiac arrhythmia, atrial fibrillation, ventricular fibrillation, atrioventricular block, ventricular tachycardia, heart failure, damage from myocardial infarction, damage from stroke, brugada syndrome, left bundle branch block, or chronic ischemia.

Also provided is a method of treating a cardiac condition characterized by impaired action potential conduction in the heart, comprising implanting in a subject in need thereof the tissue patch described in this disclosure onto the surface of a cardiac muscle of the subject, where the tissue patch is implanted on an area of the cardiac muscle having impaired action potential conduction.

Also provided are methods for treating a central nervous system (CNS) disorder, a peripheral nervous system (PNS) disorder, or a skeletal muscle disorder, comprising administering to a subject in need thereof a therapeutically effective amount of any of the nucleic acids, vectors, viruses, cells, or pharmaceutical compositions described in this disclosure. The CNS disorder, the PNS disorder, and the skeletal muscle disorder are generally characterized as having impaired action potential in one or cells of the CNS or PNS or in skeletal muscles. In some embodiments, the CNS and/or PNS disorder is a condition that is associated or results from a loss of function of a eukaryotic sodium channel. Exemplary conditions include Dravet Sydrome, Severe idoppathic generalized epilepsy of infancy, Benign familial neonatal-infantile seizures, Autism spectrum disorders, epilepsy, Brugada Syndrome, Brugada Syndrome Type 1, Ataxia, Congenital Insensitivity to Pain, or Anosmia. Exemplary skeletal muscle disorders include, but are not limited to Duchenne Muscular Dystrophy (DMD), Becker's Muscular Dystrophy (BMD), Congenital Muscular Dystrophy (CMD), and Limb-Girdle Muscular Dystrophy, Myotonic dystrophy type 1 and type 2, and Emery-Dreifuss muscular dystrophy.

Aspects of the disclosure relate to a method of increasing the conduction of a cell comprising introducing any of the nucleic acids, vectors, viruses, cells, or pharmaceutical compositions described in this disclosure, where introduction of the nucleic acid, the vector, the virus, or the pharmaceutical composition induces the expression of the sodium channel polypeptide, thereby increasing conduction of the cell.

Also provided is a method of producing a tissue patch comprising (i) seeding a plurality of any of the cells described in this disclosure on a solid support, thereby forming a cell-seeded construct, and (ii) culturing the cell-seeded construct in a culture medium for a period of time, thereby producing the tissue patch.

## BRIEF DESCRIPTION OF THE DRAWINGS

The present application includes the following figures. The figures are intended to illustrate certain embodiments and/or features of the compositions and methods, and to supplement any description(s) of the compositions and methods. The figures do not limit the scope of the compositions and methods, unless the written description expressly indicates that such is the case.

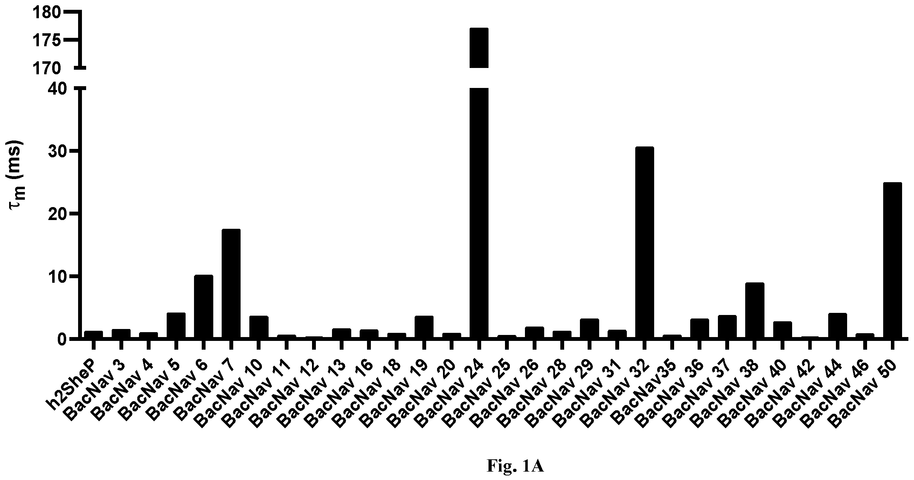

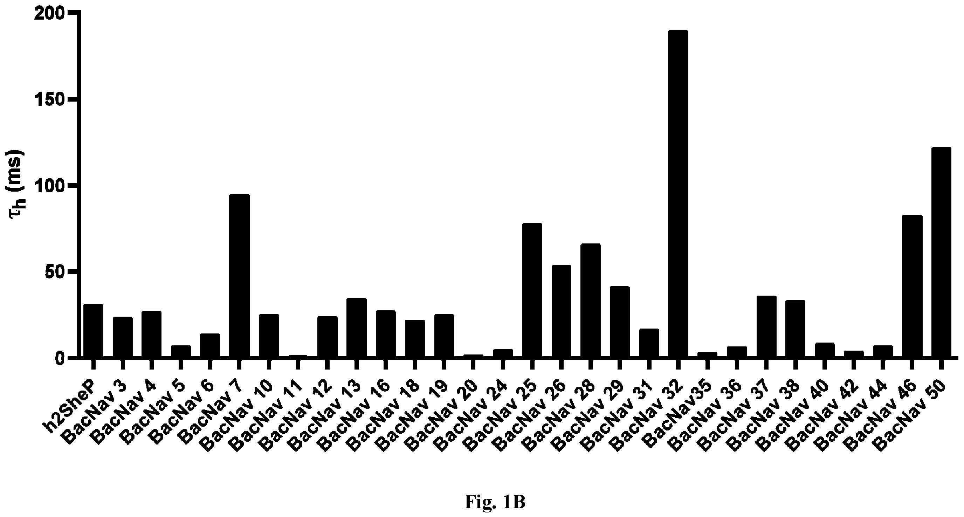

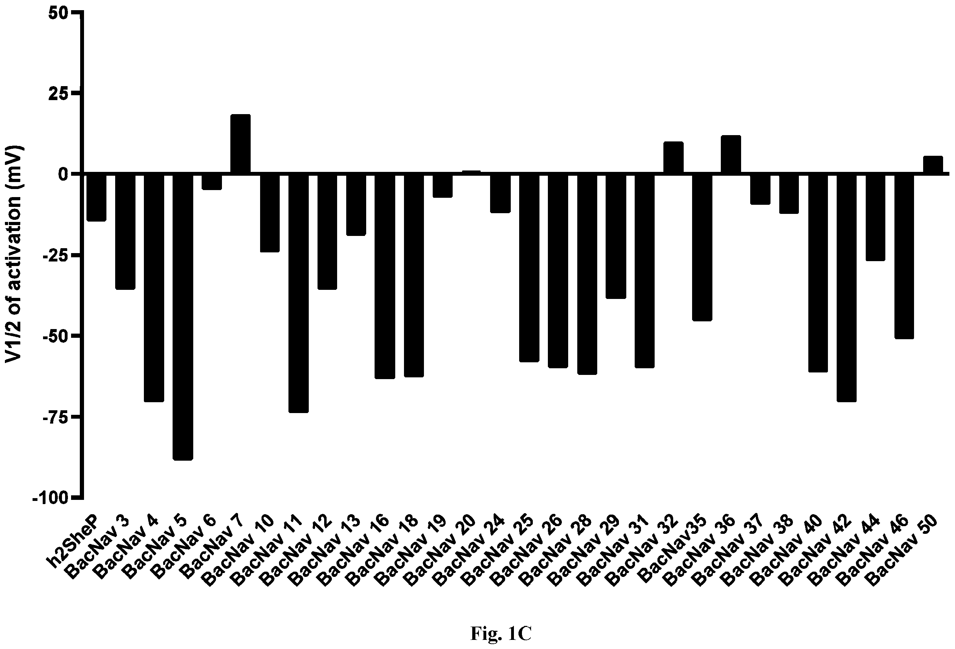

Fig. 1A shows bar graph illustrating gating kinetics indicated by lowest time constants of activation ($\tau_m$) according to certain aspects of this disclosure. Fig. 1B shows bar graph illustrating gating kinetics indicated by lowest time constants of inactivation ($\tau_h$). Fig. 1C shows bar graph illustrating gating kinetics indicated by Mid-points ($V_{1/2}$) of Boltzmann equation fitting of the steady-state values of the activation gating variables as a function of V. Electrophysiological data were obtained at 25 °C.

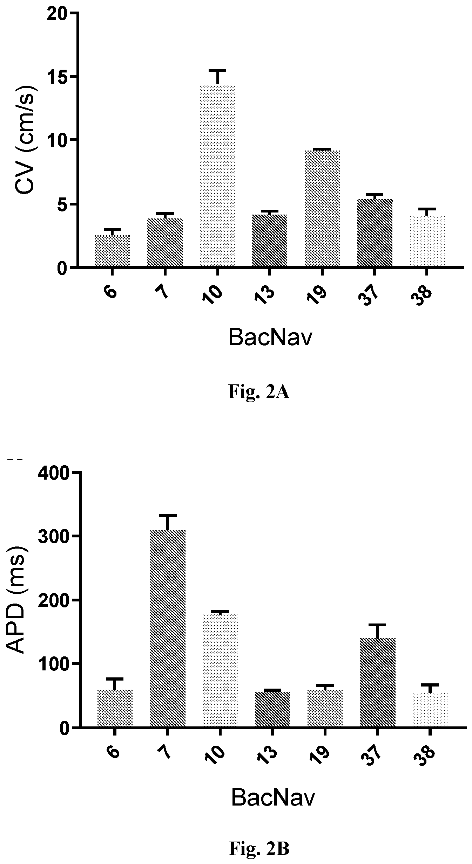

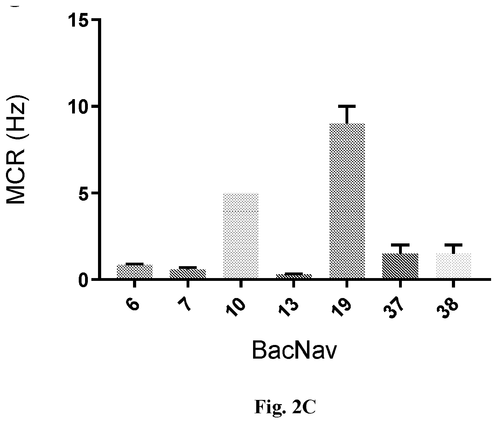

Fig. 2A shows bar graph illustrating conduction velocity (CV, n=2-6) of each BacNa$_v$ according to certain aspects of this disclosure. Fig. 2B shows bar graph illustrating action potential duration (APDso, n=2-6) of each BacNa$_v$. Fig. 2C shows bar graph illustrating maximum capture rate (MCR, n=2-6) of each BacNa$_v$. Error bars indicate s.e.m. All screening results of BacNa$_v$ orthologs in electrically stimulated monolayers of HEK 293T cells transduced with CMV-BacNav-T2A-eGFP lentiviruses and co-expressed with Kir2.1 and Cx43. All electrophysiological data obtained at 37 °C.

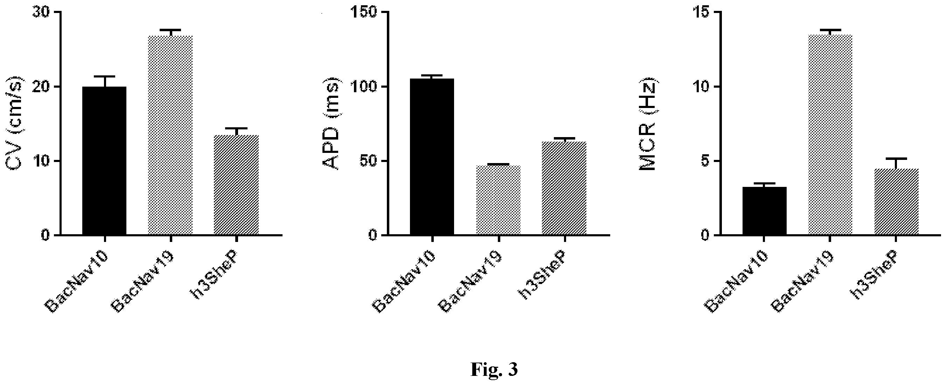

Fig. 3 shows electrophysiological properties for new fast-firing BacNa$_v$ channels and previously characterized NavSheP D60A via optical mapping in electrically stimulated monolayers of HEK 293T cells transduced with CMV-BacNav-T2A-eGFP lentiviruses and co-expressed with Kir2.1 and Cx43 according to certain aspects of this disclosure. With optimized cell seeding density and lentivirus titer, two orthologs (BacNav 10 and BacNav 19) led to faster conduction speed than human codon optimized sequence of NavSheP D60A (h3SheP). Left panel shows bar graph illustrating conduction velocity (CV, n=6-8). Center panel shows bar graph illustrating action potential duration (APD$_{80}$, n=6-8). Right panel shows bar graph illustrating maximum capture rate (MCR, n=6-8). Error bars indicate s.e.m. All electrophysiological data obtained at 37°C.

DETAILED DESCRIPTION

1. Introduction

In cardiac muscle cells sodium channels are primarily responsible for generating the rapid upstroke of the action potential (AP). In this manner sodium channels are essential to the initiation and conduction of electrical signals, and the proper function of sodium channels is therefore necessary for normal function of the heart. Reduced sodium current density and slow action potential conduction can arise from altered extracellular environment, cell morphology, or channel regulation that occur in conditions such as myocardial infarction, heart failure, and cardiac ischemia. In particular, these conditions lead to damage of cardiac tissue and the development of fibrosis, characterized by excessive fibroblast proliferation. Increased amounts of fibroblasts can separate cardiac muscle cells causing slow or discontinuous conduction. Further, genetic mutations that result in loss of function in voltage-gated sodium channels (VGSCs) can cause reduced tissue excitability, leading to various cardiac disorders. Consequently, therapies for cardiac conditions could greatly benefit from approaches that enhance electrical excitability and AP conduction in the heart via delivery of functional VGSCs. However, gene-based therapies involving VGSCs are largely hampered by the inability to stably express mammalian channels using viral delivery methods as the genes encoding the VGSCs are too large (>6 kb) to be efficiently incorporated into viral vectors.

The inventors have developed compositions and methods for treatment of cardiac conditions, *inter alia,* by introducing one or more bacterial sodium channels (BacNavs) into the heart tissue of a subject in need thereof. These BacNavs are encoded by genes that are only approximately 0.7-0.9 kb in size, approximately one-eighth to one-tenth the size of their mammalian counterparts, and thus are well-suited for gene therapy applications. In particular, the inventors have discovered that the BacNavs described herein exhibit fast conduction speed, comparable to that of mammalian sodium channels, and thus are particularly suitable for treating cardiac conditions characterized by sodium channel malfunction.

Aspects of the disclosure relate to nucleic acids comprising a nucleotide sequence encoding a bacterial sodium channel polypeptide, where the nucleotide sequence is operatively linked to a heterologous promoter. In some aspects, a viral vector may be used to deliver the bacterial sodium channels to a subject in a gene therapy application. In some approaches, gene therapy involves delivering bacterial sodium channels into cardiac cells of a mammalian subject using viral vectors described herein. In one embodiment, the method disclosed herein comprises administering the viral vectors to a subject, where the administration results in expression of bacterial sodium channels in the cardiac cells of the subject. In some embodiments, the subject in need of treatment has a cardiac condition characterized by impaired action potential conduction in the heart. In one aspect, the cardiac condition is one or more of cardiac arrhythmia, atrial fibrillation, ventricular fibrillation, atrioventricular block, ventricular tachycardia, heart failure, damage from myocardial infarction, damage from stroke, brugada syndrome, left bundle branch block, or chronic ischemia. Cells comprising a nucleic acid comprising a nucleotide sequence encoding a bacterial sodium channel polypeptide as described herein are also provided, as are pharmaceutical compositions and associated methods. In one embodiment, the compositions described herein may be used to increase conduction of a mammalian cell. Further provided is a tissue patch. The tissue patch includes a plurality of cells comprising the nucleic acid as described herein. The tissue patch may be used as an implant for treating a cardiac condition in a subject. In some aspects, the tissue patch is implanted onto the surface of a cardiac muscle of the subject, wherein the tissue patch is implanted on an area of the cardiac muscle having impaired action potential conduction. The cardiac condition may be one or more of cardiac arrhythmia, atrial fibrillation, ventricular fibrillation, atrioventricular block, ventricular tachycardia, heart failure, damage from myocardial infarction, damage from stroke, brugada syndrome, left bundle branch block, or chronic ischemia.

The following description recites various aspects and embodiments of the present compositions and methods. No particular embodiment is intended to define the scope of the compositions and methods. Rather, the embodiments merely provide non-limiting examples of various compositions and methods that are at least included within the scope of the disclosed compositions and methods. The description is to be read from the perspective of one of ordinary skill in the art; therefore, information well known to the skilled artisan is not necessarily included.

Articles "a" and "an" are used herein to refer to one or to more than one (i.e. at least one) of the grammatical object of the article. By way of example, "an element" means at least one element and can include more than one element.

"About" is used to provide flexibility to a numerical range endpoint by providing that a given value may be "slightly above" or "slightly below" the endpoint without affecting the desired result.

The use herein of the terms "including," "comprising," or "having," and variations thereof, is meant to encompass the elements listed thereafter and equivalents thereof as well as additional elements. Embodiments recited as "including," "comprising," or "having" certain elements are also contemplated as "consisting essentially of and "consisting of those certain elements. As used herein, "and/or" refers to and encompasses any and all possible combinations of one or more of the associated listed items, as well as the lack of combinations where interpreted in the alternative ("or").

As used herein, the transitional phrase "consisting essentially of" (and grammatical variants) is to be interpreted as encompassing the recited materials or steps "and those that do not materially affect the basic and novel characteristic(s)" of the claimed invention. See, In re Herz, 537 F.2d 549, 551-52, 190 U.S.P.Q. 461, 463 (CCPA 1976) (emphasis in the original); see also MPEP §2111.03. Thus, the term "consisting essentially of" as used herein should not be interpreted as equivalent to "comprising."

Recitation of ranges of values herein are merely intended to serve as a shorthand method of referring individually to each separate value falling within the range, unless otherwise-Indicated herein, and each separate value is incorporated into the specification as if it were individually recited herein. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this disclosure.

Unless otherwise indicated, nucleotide sequences are presented 5' to 3'.

## 2. Compositions

### 2.1 Nucleic acids and Polypeptides

Provided herein is a nucleic acid comprising a nucleotide sequence encoding a sodium channel polypeptide comprising an amino acid sequence having at least 90% identity to the sequence of any one of SEQ ID NOs: 1-29, where the nucleotide sequence is operatively linked to a heterologous promoter. Also provided herein is a nucleotide sequence encoding a sodium channel polypeptide comprising an amino acid sequence having at least 90% identity to the sequence of any one of SEQ ID NOs: 1-29, where the nucleotide sequence is not a naturally occurring sequence encoding the sodium channel polypeptide.

Voltage-gated ion channels (VGICs) are transmembrane proteins that generally share a conserved architecture in which four subunits or homologous domains create a central ion-conducting pore domain (PD) surrounded by four voltage sensor domains (VSDs). Eukaryotic sodium channels ($Na_v$) have a large pore-forming alpha subunit and auxiliary subunits. The alpha subunit is composed of a single polypeptide chain comprising four linearly connected homologous transmembrane domains (Domains I-IV). Transmembrane segments S1-S4 form the VSD that regulates the channel opening upon depolarization of membrane potentials. The S4 places highly conserved arginine residues within the membrane electric field that undergo outward movement upon depolarization, giving rise to the phenomena of the "gating currents". The VSD of one subunit packs alongside the PD of the neighboring subunit. Transmembrane segments S5-S6 form the PD that houses the selectivity filter (SF), which defines the ion selectivity properties of the channel. The intracellular loops that connect the $Na_v$ transmembrane domains have important roles in channel regulation. VSDs are connected to the pore domain through connecting helices S4-S5 that constitutes a hinge region responsible for opening the pore to allow ion flux.

Bacterial sodium channels ($BacNa_v$s), rather than having the twenty-four transmembrane segment architecture of eukaryotic Navs, are built from a six transmembrane segment architecture comprising a VSD and PD that assembles into homotetramers. While smaller, $BacNa_v$s share this conserved architecture. The first four transmembrane segments (S1-S4) of $BacNa_v$ form the voltage-sensing domain (VSD) while S5-S6 form the pore-lining domain (PD). These two domains are coupled via the S4-S5 linker, which provides the mechanism to opening/closing of the pore in response to changes in membrane voltage. The selectivity filter is flanked between the P1 and P2 helices in a helix-loop-helix motif. Sodium conduction of $BacNa_v$ is achieved via a multi-ion mechanism, whereby four glutamic acid residues of the selectivity filter in each of S1-S4 (TL**E**SWSM) serve as a high-field-strength (HFS) site that favors conduction of small cations whereas the adjacent backbone carbonyls (**TL**ESWSM) form the central and inner coordination sites. Selected ions then pass through the central cavity to the intracellular gate, which opens or closes in response to slight rotations of a single residue in the middle of S6 that faces directly toward the S4-S5 linker. As membrane potential is depolarized, change in electric field across the membrane induces an "outward" movement of the S4 segment, which then triggers a conformational change in the S4-S5 linker. This in turn shifts the positions of S5 and S6 helices, inducing a bend in the middle of S6 (hinge), which leads to opening of the intracellular gate. In $BacNa_v$, this "hinge" corresponds to a glycine residue, or other amino acid residue depending on orthologs, which facilitates channel opening due to its flexibility. Selected ions then pass through the central cavity to the intracellular gate, which opens or closes in response to slight rotations of a single residue in the middle of S6 that faces directly toward the S4-S5 linker. Beneath this intracellular gate is the CTD, comprising of a neck region and a distal coiled-coil domain, which has been suggested to play various roles in channel assembly, stability, and gating. The CTD sequences are highly diverse among the $BacNa_v$ superfamily, and are suggested to be responsible for the vast diversity of voltage dependencies of gating among different $BacNa_v$ orthologs. The cytoplasmic domain (CTD) that follows the pore-lining S6 transmembrane helix has two domains: a membrane proximal region termed the 'neck' region and a C-terminal coiled-coil domain (CCD). An extensive discussion of the features of $BacNa_v$s is provided in Payandeh, J. and Minor, D.L., J. Mol. Biol. 427(1):3-30 (2015).

In some embodiments, the sodium channel polypeptide is a prokaryotic sodium channel. In some embodiments, the sodium channel polypeptide is a bacterial sodium channel (BacNav).

In some embodiments, the sodium channel polypeptide comprises an amino acid sequence of any one of SEQ ID NOs: 1-29. In a certain embodiment, the sodium channel polypeptide comprises an amino acid sequence of any one of SEQ ID NO: 6. In a certain embodiment, the sodium channel polypeptide comprises an amino acid sequence of any one of SEQ ID NO: 12. The amino acid sequences of SEQ ID NOs: 1-29 are set forth in Table 1. In each polypeptide sequence, the amino acid residues of the sodium channel selectivity filter are underlined, the amino acid residues of the coiled-coil region are italicized, and the amino acid residues of the hinge region are bolded, underlined, and italicized.

Table 1. Amino acid sequences of sodium channel polypeptides

| Polypeptide | Amino acid sequence | UniProt/ SwissProt DB Entry No | Species | SEQ ID NO |
|---|---|---|---|---|
| BacNav 3 | MQATPVETSTRRRLQQLIEQPAVQR-SILLLIVINAAILGMQTSPALVASWGELLRVLD MLILGVFVVEIAARIYVHRAAFFRDPWSLFDFT VVAIALVPASGPFSVLRALRVLRVMRMVTMV PSMRRVVGALLSAIPGLGSIAMVLALVFYVSA VIATGLFGADFPEWFGNLGRSIYTLFQVM_TLES_ _WSM_GIVRPLMDVFPYAWVFFIPFILI_A_TFTMLN LFIAIIVNAMQTVTDAEHEATQASIEAAREHI*EA DLHEEVRALRGEIAELKDLLRGQARR* | A0A098FXJ6 | Pseudomonas xanthomarina | 1 |
| BacNav 4 | MAGTPPLRQRLHAFLKQPYVERTIIALILINAVI LGLETSPATMSTVGHLLVAVDQAILAIFVVEIA LRIYVHRLDFWRDPWSIFDFTVVAIALVPAAG PFAVLRALRVLRVLRLLTMVPSMRKVVGALL AAIPGLGSIALVLLILYYVFAVIATNLFASDYPE WFGSIGLSLYTLFQIM_TLESWSM_GIVRPVMEQ FPFAWAFFIPFILV_A_TFTMLNLFIAIIVNAMQSY SESEHQETVQAIQITQEHI*EADLHSEVRGLRAEIR ELKGLLMGQTSKGG* | A0A2N6GGE0 | Sedimenticola sp. | 2 |
| BacNav 5 | MVEVILRDLDPASLRARAGRWIESPRVQHAIV FLIVINAIILGLETSPAVMERVGPALRLADSLIL GVFVVEIAIKLFAYRLRFFTIAWNVFDFIVVGIA LIPASGPLAVLRALRVLRVLRLISMVPKLRFIVE ALLHAVPGIASIAGLMLLLFYVFAVMATGLFG QQFPDWFGSIGASMYTLFQIM_TLESWSM_GIVR PVMELYPHAWLFFVPFILI_A_TFTMLNLFIGIIVD TMQTMHEAEHSRDRSLIEQRIEAE*GGAIEDEIR GLRAEIRELKAALDRGRP* | A0A1Z4VN78 | Thiohalobacter thiocyanaticus | 3 |
| BacNav 6 | MRNQVKALVTSRTWEFCIIGIIVLNAVTLGLET SHAVMNSIGPALVLIDQIILGIFVIELALRLFAH GAKFFRDPWSLFDFAIVAIALLPSNGPLAVLRS LRILRVLRLISVVPSLRRVIGGLIAALPGMGSIV | A0A222FCR4 | Labrenzia sp. VG12 | 4 |

| Polypeptide | Amino acid sequence | UniProt/ SwissProt DB Entry No | Species | SEQ ID NO |
|---|---|---|---|---|
| | VLMALVFYVFGVMATKLFGASFPEWFGDLGA SLYTLFQTLESWSMIMGIVRPVMEQYPYSWLF FVPFILCTAFTVLNLFIGIIVSAMQEEHEAEADA NRQAIHDETGLILEEVKAMRAELSQLRAEVERTR A | | | |
| BacNav 7 | MAEHEGLRKRMGVWIESAAIQRLIIILILVNALI LGLQTSVTLMTHFGSYLIWADRLILSVFVIELLI KLFALDRRFFKNPWNVFDLVVVSLALIPSTGPL SVLRVLRLLRLVSLLPKLRFVVEALLRAIPGIISI IGLLGLLYYVFAVIATGLFGQQFPDWFGTLGK SMYTLFQIMTLESWSMGIARPVLESYPYAWLF FIPFILVATFTILNLFIAIIVSTMQSMQEEQRAIE QQAISKTLHDDSAQIHADLLRTESENQQLREELR AVREDIRALRELLAKQNP | G4E512 | Thiorhodospira sibirica ATCC 700588 | 5 |
| BacNav 10 | MRETGVTEYTGLRGRLHAFIAHPKVQAGILAL IVVNAILLGLETAPQIMAEAGGLIRLADRVILA VFVVEIAIRLFVHRGRFFRDPWSLFDFAVVAIA LVPATGPFSVLRALRVLRVLRVLTIVPSMRRV VGALLGAVPGLLSIGMVLVLIYYVCAVIATNL FGPVFPDWFGNIGRSFYTLFQIMTLESWSMGIS RPVMEEFPYAWAFFIPFILVATFTMLNLFIGVIV DAMQTVSEAEHADTLDALDRTQDHIEADVHA EVRALRDEIRSLRTLLETRSER | A0A2A3LK12 | Xanthomonada ceae bacterium NML93-0399 | 6 |
| BacNav 11 | MPSSTAVVTNSMRQRLKTFIENSTIQRILLALIL INAVILGLETSPDVMTTAGSFLMALDKAILAVF VIELTIRLLVHRFAFFKDGWNVFDFIVVGIALV PASGPFAVLRALRVLRVLRVLTFVPSMRKIVG ALIKSLNGMLSIAMVLGLVYYVAAVMVTKLF GEAFPEWFGSLGASLYTLFQVMTLESWSMGIA RPVMEAFPYAWAFFIPFILLATFTMLNLFIAVIV NAVQTMHDDEHKDELDAEKATQQQLLEQMQQ LQLELKALRRDINKPQE | A0A1M5B5U9 | Marinomonas polaris DSM 16579 | 7 |

(continued)

| Polypeptide | Amino acid sequence | UniProt/ SwissProt DB Entry No | Species | SEQ ID NO |
|---|---|---|---|---|
| BacNav 12 | MKALLERALAHPFTERFVLAVILINAITLGLET DARVMARFGEWLLVLDKAALVIFCIELAAKL YVRGLRFFRDPWNVFDFVVVAIALLPASGPLS VLRALRILRVLRLTIVPSLKRVVGALLGALPG MASIVMLLVLIFYVGAVMSTKLFGEAFPEWFG SVADSFYTLFQVMTLESWSMGIVRPVMEVYP YSWLFFIPFILATSFTALNLFIGVVVSAMQAEID ADRERVVEEAVAKGEEPLVEEVRALRAEIARLS QRFEGTDRPA | A0A1G6SVE7 | Aquimonas voraii | 8 |
| BacNav 13 | MQERLQHLFRSRRWEQFIIGLIVVNAIFLGLET SDAVMRNIGGLLLAFDTAILAVFVIEIVGRIYA FRGAFFRDPWSIFDFTVVGIALLPATGPLTVLR ALRILRVLRLISVIPSLRRVIGGLIAALPGMGSII ALLAITFYIFAVMATKLFGDTFPQWFGDMGAS IYTLFQVMTLESWSMGIVRPVMEVHPYAWLFF VPFILSTTYAVLNLFIGVIVSAMQGEHFASIDAE HEKRHNENTEILAEVKALRAELAEMRAAKAEG | Q0FZH3 | Fulvimarina pelagi HTCC2506 | 9 |
| BacNav 16 | MTNSLPDAPTGLQARIINLVEQNWFGRFILTLI LINAVLIGMFTSASLMAQYGPLIVSIDKLILIG IFVLELLRIFAYRSEFFKDAWSLFDFAVVAIAL IPASGPLAVLRSLRVLRVLRVLSIVPSMKRVVS ALLGSMPGLASIGMVLVLIYYVFAVIATKIFGT AFPEWFGTIGASFYTLFQVMTLESWSMGISRP VMEMFPYAWLFFIPFILVATFTMLNLFIAIIVNT MQTFSDEEHALERALDKQAQDQFQQQMHEEL KTIRQELQQLQAILRSSPGMQPHAPSNPDQPSD | A0A1B7UGQ3 | Rheinheimera sp. SA_1 | 10 |
| BacNav 18 | MHAKLKLLIENPVTQRIISLIVINAVLLGLETS GAIMAAAGTYIVLLDKAILGVFVVEIVSRIYIY RLNFWKDPWSLFDFAVVSIAIVPSSGAFSVLRA LRVLRLLTMVPSMRRVVGALLSAVPGLISI AMVLLIIYYVFAVISTNLFAEQYPQWFGSLGLS LYTLFQIMTLESWSMGIARPVMETFPYAWAFF IPFILVATFTMLNLFIAIIVNAMQSFNEEERKETI DAVNVLDNDIQGELKLLRQEIRELRQFLTNKAL | A0A1N7MWC6 | Neptunomonas antarctica | 11 |

| Polypeptide | Amino acid sequence | UniProt/ SwissProt DB Entry No | Species | SEQ ID NO |
|---|---|---|---|---|
| BacNav 19 | MDRLRAFITSRRTEYFITGLIIINAITLGLETDAT IAANFGDALHIFDQFILGVFVVELMLRILVHRT SFFKDPWNLFDFLIVTIALLPSSGSLSVLRALRI LRVLRLITFVPTLRRVVGALIGALPGMGSIILL MALVYYVFAVMATKLFGETFPQWFGTLGESA YTLFQIM<u>TLESWSM</u>GIVRPVMEQFPLAWLFFV PFILS*T*TFTVLNLFIGIIVAAMQTEHDAEAELE*R QALHEENVNVLEEVKALRKE*ISTLHDLVKTNRA | A0A2A4PC72 | Rhizobiales bacterium | 12 |
| BacNav 20 | MSAWLRSIVDDPRAERVIMVLIVINAVILGLET SETIMASHGWLLETLDRAILAVFVVEIAARIIA YRGAYFRDPWNVFDFIVVAIALVPATETFSVL RALRVLRVLRLVTRVPSLRRVVGGLITALPGM GSVVALLSLLFYVFAVMATKLFGEQFPDWFGS IGASAYSLFQIM<u>TLESWSM</u>GIVRPVMETYPYA WAFFVPFIVI*T*TFAVLNLFIGIV*VNAMQAEHEKA VNEERAAERD*MIHDETAPLVEEIKALRVEMAA LRQRIENPAVRPPE | A0A1G6DQH3 | Bauldia litoralis | 13 |
| BacNav 24 | MSSREQVGAWIESTRIQRIIIVLILVNAVTLGLE TSSRIMASYGGFLHLLDRVILAAFVAEILLKLF AHGLGFFRRGWNLFDFTVIAIALIPASGPLAVL RALRVLRVLRLVSVSPRLRFVVEALLKALPGIA SIASLMLLLFYVAAVIATGLFGTGFPQWFGTLG RSMYTLFQIM<u>TLESWSM</u>GIVRPVMDVHPYAW LFFIPFILI*A*TFTMLNLFIGIIVDTMQTLHDDQHA AERERIEQTVHSD*TRAVELEVRALREEIEGLRRD LAMRAKQ*S | F9U5H9 | Thiocapsa marina 5811 | 14 |

| Polypeptide | Amino acid sequence | UniProt/ SwissProt DB Entry No | Species | SEQ ID NO |
|---|---|---|---|---|
| BacNav 25 | MSSESLRYRAGVWIESKPVQNFIIALIVINAVTL GLQTSSSWMAQSGGLLLQLDNLILAVFVAEIGI KLFAFRLGFFKTGWNNFDFIVVGIALVPASGPL AVVRALRILRVLRMISMVPRLRFVVEALLHAIP GISSIGLLMLIIFYVFAVMATTLFGGDFPEWFGS IGASMYTLFQVM<u>TLESWSM</u>GIVRPVMELFPYA WLYFIPFILI*A*TFTMLNLFIGIIVDTMQTMHQAD HDEEREHIEQVVHEDT*GELANEMRQLRAELAG MRASLGK* | A0A1T2L3T4 | Solemya velesiana gill symbiont | 15 |
| BacNav 26 | MDRLRQIVTSPRTERFILALIILNAITLGLETSS WVMDRIGPVLLVLDKIVLAIFVVEVVARIAVH RLAFFKDPWSLFDFGVVAIALVPAAGPFSVLR ALRILRVLRMITIVPSLKRVVGALISALPGMGSI VLLMGLIFYVASVMATKLFGADFPQWFGSIPA SAYSLFQIM<u>TLESWSM</u>GIVRPVMEVHPYAWM FFVPFILC*T*TFTMLNLFIGIVVNAM*QAEHEEEAK AERHKLEEDLRLASEERQKAHAEDVADMAALRG ELAELRQAMTDLTTS*LTRRPG | A0A2D8I6C5 | Brevundimonas sp. | 16 |
| BacNav 28 | MSTDTLPSHGLRQRCHAFLSQPLVQHGILALIV LNAVFMGLETSASVMAEVGPWLLAVDKVILG VFVLELAVRLYVHRSAFFRDPWSLFDFAVVAI ALVPASGPFAVLRALRVLRVLRVLTIVPSMRR VVGALLSAIPGLSSIAMVLMLVFYVFAVIATHL FGQQFPDWFGHLGRSLYTLFQVM<u>TLESWSM</u>G ISRPVMEESPYAWAFFIPFILF*A*TFTMLNLFIAII VNAMQTFTESEHQATVGAVETVGQSI*EHELHA EVQSLRQEIGELKTMLRASSLA*GAFVAPNAPTSG NSG | A0A2N2TSC8 | Betaproteobact eria bacterium HGW-Betaproteobact eria-16 | 17 |

EP 4 450 514 A2

| Polypeptide | Amino acid sequence | UniProt/ SwissProt DB Entry No | Species | SEQ ID NO |
|---|---|---|---|---|
| BacNav 29 | MNATSLSLQQRTRKLVEHPRFTGTILTLIILTAII LGMETSPTVVAQWGPTLGLINNLFLAVFVVEL VLRIYAWRTRFFVDPWSLFDLIVVGISLVPASG PLAILRALRVLRVLRLVSAVPAMRKVVAALLG ALPGLGSIVVLLLLIYYVAAVIATNIFGADFPD WFGTLGRSFYTLFQIMTLESWSMGISRPVMDT FPWAWAFFIPFILIATFTMLNLFIAIIVNTMQTF HEAEQAEQQWEKDRLEQADKDYLHDQLERIQ KQLDQLSRNLEKS | A0A2E4R8B4 | Pusillimonas sp. | 18 |
| BacNav 31 | MIPSAAAGAGWRRRLADLLEAPRMQGALIALI LVNAAVLGLETSPSVMARWGGLLVRIDTAILA VFVVEIALRLVARGPRFFRDPWAVFDFVVVGI ALLPASGPFAVLRALRVLRVLRLMTFVPSMRR VVGGLLAAIPGLSSVFAVIALIFYVGSVMATKL FGGQFPEWFGSLGASAYTLFQVMTLESWSMGI ARPVMEAFPHAWAFFVPFILIATFTMLNLFIAVI VNAIQAEHAAEHETEVRDIESAVSAHADERAD ALHVEIRALRAEVARLAALLEKRG | A0A1D2TLN1 | Lautropia sp. SCN 70-15 | 19 |
| BacNav 32 | MHAVAGPRARVGAFIESDRIQRWIIALILINAA VLGLETSPTVMEHTVGPWLLVADKIILGIFVVE ILLKLFAQGWGFFRRPWNVFDFLVVGIALVPA SGPMAVLRVLRLLRLVSMMPKLRFIVEALLKA IPGILSILGLLVLLFYVFAVIATGLFGKSFPEWF GNLGQSMYTLFQVMTLESWSMGIARPVMEEY NWAWVFFVPFILIATFTILNLFIAIIVNTMQSMQ EDQQQFEHDTIEEVVHAENTQLHEDLKALRQEI RELRKEISSDRPSGPG | A0A161HHG9 | Ectothiorhodos pira sp. BSL-9 | 20 |

| Polypeptide | Amino acid sequence | UniProt/ SwissProt DB Entry No | Species | SEQ ID NO |
|---|---|---|---|---|
| BacNav 35 | MTTVETNSNGLRARVAALVERSLFQHFVTAVILVNAVTLGLETSASAMAAAGPLLIALDRIALSIFVVELALKLFAQRTRFFRDGWNIFDFIIVGIALVPAAGPFSVLRALRILRVLRLLSVVPSLRKVIASLIGALPGMGSIIAVLFLVFYVGAVLATKLFGASFPDWFGTIGGSMYSLFQIM*TLESWSM*GIVRPVMEVYPYAWIFFVPFIVM*T*SFMVLNLFIAIIVNSM*QALHEEEHNRAQDERERLAREERAAIEKRAHAERE*ATLEGVRALRAELAELRALIEARPG | A0A2N3EFQ8 | Alphaproteobac teria bacterium HGW-Alphaproteobac teria-11 | 21 |
| BacNav 36 | MNTKTITDRRQRVRRWLERPLVQQTIIALIIFNALLLGLETSPAVMAHAGGLIVAIDQAILAVFVVEIALRLYAYRAAFWRDPWSIFDFFVVAIALIPATGPLAVLRALRVLRVLRLLAMVPSMRRVVGALLVAVPGPGSIALVLLIIYYVFAVIATNLFATSYPDWFGDIGRSLYTLFQIM*TLESWSM*GIVRPVMESFSYAWAFFIPFILI*A*TFTMLNLFIAIIVNAMQMVSEADRCNAAQTLEHQSERIEGELHAE*MGRLRVEIQALRAML*SITPAPGQTKPS | A0A2N2UXD6 | Betaproteobact eria bacterium HGW-Betaproteobact eria-11 | 22 |
| BacNav 37 | MQEATLPSGQNVIERLRRLVESRRFTGFITAVILFNAVTLGLETWSYAMEVAGGLLVAIDRIVLAIFVFEMIAKLLVYRQAFFRSGWNIFDLTIVGIALVPAAGPLTVLRALRILRVLRLLSVVPQMRSVVAALIGAIPGMGSIAAVMSLIFYVGSVLATKLFGAAFPDWFGSIGASAYSLFQIM*TLESWSM*GIVRPVMEVYPWAWLFFVPFILI*T*TFAVLNLVVAVI*VNSMQTLHEAEQKEEREAERQIVHEETA*ALTGEVRAMRQELSEIKALLGAKA | A0A1E2RV77 | Methyloligella halotolerans | 23 |

(continued)

| Polypeptide | Amino acid sequence | UniProt/ SwissProt DB Entry No | Species | SEQ ID NO |
|---|---|---|---|---|
| BacNav 38 | MREKMLVLTKSREWESFILIVIIINAITLGFETSE TIMQSFAGPALRLFDRAVLVIFVVEIAIRIYAHR LRFFRDPWSIFDFTIVVISLLPASGPLQVLRALRI LRALRLLSMIPSLRRVIGGLIAALPGMGSIIVLM ALVFYIFAVIATKLYGEAFPEWFGSLGATIYSL FQIM_TLESWSM_GIVRPVMEVYPNAWLFFVPFI LS_T_AFTVLNLFIGIIVSAMQKEHEEELREEDRQ AREPE_MQELLHEVRALRSEVAALRQD_QAPRPAP TTA | A0A0D5LXK3 | Martelella endophytica | 24 |
| BacNav 40 | MTEITGTVERSLQQQVATWLQKNWVQRSLLS LILINAVILGLETAPGVMAVAGAPLMLLDKLIL AVFVLEIALRIFAYRGAFFKDAWSLFDFTVVAI ALVPASGPFAVLRALRVLRVLRVLTFVPSMKK IVGALVQSLNGMLSIAMVLGLVYYVSAVMAT KLFGEAFPEWFGNIGRTLYTLFQIM_TLESWSM_ GISRPVMEQFPYAWAFFVPFIL_A_TFTMLNLFIA VIVNAVQSMHDEEHKEE_IDAKQQLQHDLVSQM QQLQAELAALRAQ_LPPNNKVD | 19DQU4 | Alishewanella agri BL06 | 25 |
| BacNav 42 | MNSDTIVTAVPVRQRLQQFIEHGTVQRMLLAL ILLNAFTLGLETSNAVMSLAGTAIHLLDKAILA IFVLEILVRLYVHRLAFFKDAWSVFDFVVVGIA LLPASGPFSVLRALRVLRVLRVLTFVPSMKKIV GALMQSLNGMLSIAMVLGLVYYVASVMVTK LFGAAFPEWFGSLGASLYTLFQIM_TLESWSM_G IARPVMEQFPYAWLFFVPFIL_A_TFTMLNLFIAV IVNAVQSMHDAEHKTEQDAEKAT_QLQLLQQM QQLKQQLSEVQQQLRDRSN_ | A0A2E6EFX1 | Alteromonadac eae bacterium | 26 |

(continued)

| Polypeptide | Amino acid sequence | UniProt/ SwissProt DB Entry No | Species | SEQ ID NO |
|---|---|---|---|---|
| BacNav 44 | MFHTPGVNPGLRERAGRWIESGPVQRVIIALILI NAAILGLETDPDIMARIGDWLIGADRVILGVFV VEILIKLYAKGLRFFRNPWNVFDFLVVGIALIP ASGPFAVLRILRLLRLVSMIPKLRFVVEALLRAI PGIASIFGLLIILFYVFAVIATGLFAKDHPEWFG SIGRSMYTLFQVM_TLESWSM_GIARPVMETHPY AWVFFVPFILV_A_TFTILNLFIAIIVNTMQTLAEE QQKFEEKTITTVVHAES*AQLHQDLTRVESENQQ LHQDLRALREEIRALREE*LRRPG | B8GSY6 | Thio alkali vibri o sulfidiphilus (strain HL-EbGR7) | 27 |
| BacNav 46 | MNAQLLGDTTGFRAKAHQFIENPFIQNGILVLI VINAITLGLETVPAAMQRFSNIIHTLDLVILSVF VLELLIRLYVYREKYFNDPWRAFDFVVVSIAL VPATGQLAVLRALRVLRVLRIITIVPSMRRVVG ALLSAIPGLTSIALVLGLIYYVFAVIATNLFAAE FPEWFGHLGRSFYTLFQIM_TLESWSM_GIARPV METFPYAWAFFIPFILV_A_TFTMLNLFIAIIVNAM QTFTEQEKQGTVEAVNEARDHI*EEDMHTEMRA LRREIAELKSMIRGQYRQ*DP | A0A1Y0IGL1 | Oleiphilus messinensis | 28 |
| BacNav 50 | MANLTDSAGNPGHRAWLREWVESAPFRYTVL VIIFINAIVLGLETEASVIAEVGDMLHLIDKIILW IFVVELILRMYAHGPRFFLDPWGVFDFIIVAIAL FPASFEFSVLRALRILRALRLISGVPRMRRVVE ALLRAVPGIGSVAALLLLVFYVFSVIATKLFGT AFPQWFGTIGESMYSLFQIM_TLESWSM_GIVRP VMEEYPEAWAFFVPFIII_S_SFTVLNLFIAIIVDSM QTLHADEEERTVERIETIVDED*TQLVSDEIARLR AEIRDLRSELNGRKS* | A0A2E6GQK6 | Rhodospirillace ae bacterium | 29 |

EP 4 450 514 A2

As used throughout, the term "nucleic acid" or "nucleotide" refers to deoxyribonucleic acids (DNA) or ribonucleic acids (RNA) and polymers thereof in either single- or double-stranded form. It is understood that when an RNA is described, its corresponding DNA is also described, wherein uridine is represented as thymidine. Unless specifically limited, the term encompasses nucleic acids containing known analogues of natural nucleotides that have similar properties as the reference nucleic acid and are metabolized in a manner similar to naturally occurring nucleotides. A nucleic acid sequence can comprise combinations of deoxyribonucleic acids and ribonucleic acids. Such deoxyribonucleic acids and ribonucleic acids include both naturally occurring molecules and synthetic analogues. The polynucleotides also encompass all forms of sequences including, but not limited to, single-stranded forms, double-stranded forms, hairpins, stem-and-loop structures, and the like. Unless otherwise indicated, a particular nucleic acid sequence also implicitly encompasses modified variants thereof, alleles, orthologs, SNPs, and complementary sequences as well as the sequence explicitly indicated.

The terms "polypeptide," "peptide," and "protein" are used interchangeably herein to refer to a polymer of amino acid residues. As used herein the term "amino acid" refers to naturally occurring and non-natural synthetic amino acids, as well as amino acid analogs and amino acid mimetics that function in a manner similar to the naturally occurring amino acids. Naturally occurring amino acids are those encoded by the genetic code. Amino acids can be referred to herein by either their commonly known three-letter symbols or by the one-letter symbols recommended by the IUPAC-IUB Biochemical Nomenclature Commission. Amino acids may include, for example, Gly, Ala, Val, Ile, Leu, Met, Phe, Tyr, Trp, Cys, Pro, Arg, His, Lys, Asp, Glu, Ser, Thr, Asn, and Gln. Amino acids include the side chain and polypeptide backbone portions. Unnatural amino acids (that is, those that are not naturally found in proteins) are also known in the art, as set forth in, for example, Zhang et al. "Protein engineering with unnatural amino acids," Curr. Opin. Struct. Biol. 23(4): 581-587 (2013); Xie et la. "Adding amino acids to the genetic repertoire," 9(6): 548-54 (2005)); and all references cited therein.

In some embodiments, the nucleotide sequence encoding a sodium channel polypeptide comprises an amino acid sequence having at least 90% identity to the sequence of any one of SEQ ID NOs: 1-29. In some embodiments, the nucleotide sequence encoding a sodium channel polypeptide comprises an amino acid sequence sharing at least 90%, at least 91%, at least 92%, at least 93%, at least 94%, at least 95%, at least 96%, at least 97%, at least 98%, at least 99%, or more percent sequence identity with the sequence of any one of SEQ ID NOs: 1-29. In some instances, the nucleotide sequence is not a naturally occurring sequence encoding the sodium channel polypeptide.

The term "identity" or "substantial identity", as used in the context of an amino acid or nucleotide sequence described herein, refers to a sequence that has at least 60% sequence identity to a reference sequence. Alternatively, percent identity can be any integer from 60% to 100%. Exemplary embodiments include at least: 60%, 65%, 70%, 75%, 80%, 85%, 90%, 91%, 92%, 93%, 94%, 95%, 96%, 97%, 98%, or 99%, as compared to a reference sequence using the programs described herein; preferably BLAST using standard parameters, as described below.

For sequence comparison, typically one sequence acts as a reference sequence to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, about 20 to 50, about 20 to 100, about 50 to about 200 or about 100 to about 150, in which a sequence may be compared to a reference sequence of the same number of contiguous positions after the two sequences are optimally aligned. Methods of alignment of sequences for comparison are well-known in the art. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85: 2444 (1988), by computerized implementations of these algorithms (e.g., BLAST), or by manual alignment and visual inspection.

Algorithms that are suitable for determining percent sequence identity and sequence similarity are the BLAST and BLAST 2.0 algorithms, which are described in Altschul et al. (1990) J. Mol. Biol. 215: 403-410 and Altschul et al. (1977) Nucleic Acids Res. 25: 3389-3402, respectively. Software for performing BLAST analyses is publicly available through the National Center for Biotechnology Information (NCBI) web site. The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits acts as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands.

The BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.01, more preferably less than about 10-5, and most preferably less than about 10-20.

In some embodiments, the sodium channel polypeptide comprises an amino acid sequence with one or more substitution mutations that improve gating or other properties. For example, the amino acid sequence may include substitution mutations that alter the voltage dependencies of gating. In some embodiments, the amino acid sequence includes one or more substitution mutations at the extracellular negative charge clusters. In some embodiments, the amino acid sequence includes one or more substitution mutations at the intracellular negative charge clusters. In some embodiments, the amino acid sequence includes one or more substitution mutations at the S4 gating region. In some embodiments, the amino acid sequence includes one or more substitution mutations at the activation gate region. In some embodiments, the amino acid sequence includes one or more substitution mutations at the proximal "neck" region located between the activation gate and the four-helix bundle region of the C-terminal domain (CTD).

In some embodiments, the amino acid sequence includes one or more substitution mutations that increase gating kinetics. In some embodiments, the amino acid sequence includes one or more substitution mutations at the hinge region. In some embodiments, the amino acid sequence includes a hydrophilic substitution at the S2 transmembrane region. Hydrophobic amino acids include, for example, Ala, Val, Ile, Leu, Met, Phe, Tyr, Trp, Cys, and Pro. Hydrophilic amino acids include, for example, Arg, His, Lys, Asp, Glu, Ser, Thr, Asn, and Gln.

In some embodiments, the substitution mutation is in one or more of any of the amino acid position indicated for the sodium channel polypeptides as shown in Table 2 (N/A in Table 2 indicates no corresponding position is identified).

Table 2. Position for substitution mutations for each sodium channel polypeptide

| Polypeptide | Mutation sites | | | | | |
|---|---|---|---|---|---|---|
| | Extracellular negative charge cluster | S2 transmembrane region | Intracellular negative charge cluster | S4 gating region | Hinge region | Activation gate region |
| BacNav 3 | Q41, D58 | L62 | E68, D89 | R108, R111, R114, R117 | A214 | M230 |
| BacNav 4 | E38, D55 | L59 | E65, D86 | R105, R108, R111, R114 | A211 | M227 |
| BacNav 5 | E45, D62 | L66 | E72, D93 | R112, R115, R118, R121 | A218 | M234 |
| BacNav 6 | E32, D49 | L53 | E59, D80 | R99, R102, R105, R108 | T205 | M221 |
| BacNav 7 | Q38, D55 | L59 | E65, D86 | R105, R108, N/A, R111 | A208 | M224 |

(continued)

| Polypeptide | Mutation sites | | | | | |
|---|---|---|---|---|---|---|
| | Extracellular negative charge cluster | S2 transmembrane region | Intracellular negative charge cluster | S4 gating region | Hinge region | Activation gate region |
| BacNav 10 | E43, D60 | L64 | E70, D91 | R110, R113, R116, R119 | A216 | M232 |
| BacNav 11 | E43, D60 | L64 | E70, D91 | R110, R113, R116, R119 | A216 | V232 |
| BacNav 12 | E32, D49 | L53 | E59, D80 | R99, R102, R105, R108 | T205 | M221 |
| BacNav 13 | E32, D49 | L53 | E59, D80 | R99, R102, R105, R108 | T205 | M221 |
| BacNav 16 | E43, D60 | L64 | E70, D91 | R110, R113, R116, R119 | A216 | M232 |
| BacNav 18 | E32, D49 | L53 | E59, D80 | R99, R102, R105, R108 | A205 | M221 |
| BacNav 19 | E31, D48 | L52 | E58, D79 | R98, R101, R104, R107 | T204 | M220 |
| BacNav 20 | E32, D49 | L53 | E59, D80 | R99, R102, R105, R108 | T205 | M221 |
| BacNav 24 | E34, D51 | L55 | E61, D82 | R101, R104, R107, R110 | A207 | M223 |
| BacNav 25 | Q37, D54 | L58 | E64, D85 | R104, R107, R110, R113 | A210 | M226 |
| BacNav 26 | E31, D48 | L52 | E58, D79 | R98, R101, R104, R107 | T204 | M220 |

(continued)

| Polypeptide | Mutation sites | | | | | |
|---|---|---|---|---|---|---|
| | Extracellular negative charge cluster | S2 transmembrane region | Intracellular negative charge cluster | S4 gating region | Hinge region | Activation gate region |
| BacNav 28 | E42, D59 | L63 | E69, D90 | R109, R112, R115, R118 | A215 | M231 |
| BacNav 29 | E39, N56 | L60 | E66, D87 | R106, R109, R112, R115 | A212 | M228 |
| BacNav 31 | E42, D59 | L63 | E69, D80 | R109, R112, R115, R118 | A215 | 1231 |
| BacNav 32 | E38, D56 | L60 | E66, D87 | R106, R109, N/A, R115 | A209 | M225 |
| BacNav 35 | E42, D59 | L63 | E69, D90 | R109, R112, R115, R118 | T215 | M231 |
| BacNav 36 | E40, D57 | L61 | E67, D88 | R107, R110, R113, R116 | A213 | M229 |
| BacNav 37 | E43, D60 | L64 | E70, D91 | R110, R113, R116, R119 | T216 | M232 |
| BacNav 38 | E32, D50 | L54 | E60, D81 | R100, R103, R106, R109 | T206 | M219 |
| BacNav 40 | E43, D60 | L64 | E70, D91 | R110, R113, R116, R119 | A216 | V232 |
| BacNav 42 | E43, D60 | L64 | E70, D91 | R110, R113, R116, R119 | A216 | V232 |
| BacNav 44 | E42, D59 | L63 | E69, D90 | R109, R112, N/A, R118 | A212 | M228 |
| BacNav 46 | E43, D60 | L64 | E70, D91 | R110, R113, R116, R119 | A216 | M232 |

(continued)

| Polypeptide | Mutation sites | | | | | |
|---|---|---|---|---|---|---|
| | Extracellular negative charge cluster | S2 transmembrane region | Intracellular negative charge cluster | S4 gating region | Hinge region | Activation gate region |
| BacNav 50 | E44, D61, | L65 | E71, D92 | R111, R114, R117, R120 | S217 | M233 |

In some embodiments, the sodium channel polypeptide comprises an amino acid sequence having at least 90% (e.g., 92%, 94%, 95%, 96%, 97%, 98%, 99%) identity to the sequence of SEQ ID NO: 6, wherein the amino acid sequence comprises one or more substitution mutations at one or more of positions E43, D60, L64, E70, D91, R110, R113, R116, R119, A216, and M232. In some embodiments, the sodium channel polypeptide comprises an amino acid sequence having at least 90% (e.g., 92%, 94%, 95%, 96%, 97%, 98%, 99%) identity to the sequence of SEQ ID NO: 6, wherein the amino acid sequence comprises a substitution mutation at position D60, and wherein the substitution mutation is substitution of amino acid aspartate with alanine, asparagine, or serine. In some embodiments, the sodium channel polypeptide comprises an amino acid sequence having at least 90% (e.g., 92%, 94%, 95%, 96%, 97%, 98%, 99%) identity to the sequence of SEQ ID NO: 12, wherein the amino acid sequence comprises one or more substitution mutations at one or more of positions E31, D48, L52, E58, D79, R98, R101, R104, R107, T204, and M220.

Also contemplated are conservative amino acid substitutions. By way of example, conservative amino acid substitutions can be made in one or more of the amino acid residues, for example, in one or more lysine residues of any of the polypeptides provided herein. One of skill in the art would know that a conservative substitution is the replacement of one amino acid residue with another that is biologically and/or chemically similar. The following eight groups each contain amino acids that are conservative substitutions for one another:

1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M).

By way of example, when an arginine to serine is mentioned, also contemplated is a conservative substitution for the serine (e.g., threonine). Nonconservative substitutions, for example, substituting a lysine with an asparagine, are also contemplated.

Modifications to any of the polypeptides or proteins provided herein are made by known methods. By way of example, modifications are made by site specific mutagenesis of nucleotides in a nucleic acid encoding the sodium channel polypeptide, thereby producing a DNA encoding the modification. Techniques for making substitution mutations at predetermined sites in DNA having a known sequence are well known. For example, M13 primer mutagenesis and PCR-based mutagenesis methods can be used to make one or more substitution mutations.

In some embodiments, nucleotide sequences are codon optimized for expression of a sodium channel or other polypeptide protein in a certain species, cell type, or tissue of interest. Codon optimization can be used to increase the rate of translation or to produce recombinant RNA transcripts having desirable properties, such as greater expression efficiency, as compared with transcripts produced using a non-optimized sequence. In particular embodiments, the nucleotide sequence encoding a sodium channel polypeptide is codon optimized for expression of the sodium channel polypeptide in mammalian cells such as, for example, human cells. Exemplary codon optimized nucleotide sequences encoding the sodium channel polypeptides as described herein include SEQ ID NOs: 30-58. Methods for codon optimization are readily available, for example, via Integrated DNA Technologies (IDT) codon optimization tool, (www.idtdna.com/CodonOpt), Genscript OptimumGene algorithm, ATUM Gene-GPS™ algorithm (described, for example, in Welch et al. (2009), "Design parameters to control synthetic gene expression in Escherichia coli,", PloS one 4, e7002), Optimizer (described, for example, in Raab et al. (2010), "The GeneOptimizer Algorithm: using a sliding window approach to cope with the vast sequence space in multiparameter DNA sequence optimization" Syst Synth Biol 4: 215, accessible free of charge at genomes.urv.es/OPTIMIZER), and GeneGPS® Expression Optimization Technology from DNA 2.0 (Newark, California).

To obtain expression of the sodium channel polypeptide, the nucleotide sequence is operatively linked to a heterologous promoter. The term "promoter" as used herein refers to an untranslated nucleic acid sequence typically upstream of a coding region that contains the binding site for RNA polymerase and initiates transcription of the DNA. The promoter may also include other elements that act as regulators of gene expression. For example, the promoter may include an enhancer region. The term "enhancer" refers to a nucleic acid sequence that can stimulate promoter activity and may be an innate element of the promoter or a heterologous element inserted to enhance the level or tissue specificity of a promoter. It is capable of functioning even when moved either upstream or downstream from the promoter. As used herein, the term "operably linked" refers to a linkage in which the promoter is contiguous with a nucleotide sequence encoding a polypeptide to control expression of the polypeptide, as well as promoter elements that act in trans or at a distance to control expression of the polypeptide. As used herein, "heterologous" in reference to a sequence is a sequence that originates from a foreign species, or, if from the same species, is substantially modified from its native form in composition and/or genomic locus by deliberate human intervention. Accordingly, a "heterologous promoter" is a promoter which is placed in juxtaposition to a gene by means of genetic manipulation (i.e., molecular biological techniques) such that transcription of that gene is directed by the linked promoter. As such, the promoter and the gene are not found in the same relationship to each other in nature.

Any suitable promoter may be used for the initiation of transcription. The promoter can be a eukaryotic or a prokaryotic promoter. In some embodiments the promoter is an inducible promoter. In some embodiments, the promoter is a constitutive promoter. In some embodiments, the promoter is a cytomegalovirus (CMV) promoter or a CMB enhancer fused to the chicken beta-actin promoter (CAG promoter). In some instances, the promoter is capable of directing expression in a bacterial cell. In some instances, the promoter is capable of directing expression in a eukaryotic cell. In some instances, the promoter is capable of directing expression in a human cell.

Inducible promoters can also be used. As used herein, the term "inducible promoter" refers to a promoter that initiates transcription of the DNA in the presence of an inducer (e.g., such as a chemical agent, light or temperature) and does not substantially initiate transcription in the absence of the inducer. Exemplary inducible promoters include, but are not limited to, tetracycline, glucocorticoid, ecdysone, streptogramins, macrolides, or doxycycline inducible promoters. In some embodiments, the promoter is a minimal interleukin-2 (IL-2) promoter (see e.g., Rivera at al. (2005), "Long-term pharmacologically regulated expression of erythropoietin in primates following AAV-mediated gene transfer," Blood, 105 (4): 1424-1430).

The nucleic acids described herein can also be under the control of a tissue-specific promoter to promote expression of the nucleic acid in specific cells, tissues, or organs. In some embodiments, the promoter is a skeletal muscle and heart specific promoter, such as a hybrid MHCK7 promoter (described, for example, in Salva et al (2007), "Design of tissue-specific regulatory cassettes for high-level rAAV-mediated expression in skeletal and cardiac muscle,." Mol Ther 15, 320-329), a CK8e promoter (described, for example, in Amoasii et al. (2017), "Single-cut genome editing restores dystrophin expression in a new mouse model of muscular dystrophy," Sci Transl Med, 9; 418), or a desmin promoter. In some embodiments, the promoter is a cardiac-specific promoter, such as a cardiac troponin T (cTnT) promoter (described, for example, in Prasad et al. (2011), "Robust cardiomyocyte-specific gene expression following systemic injection of AAV: in vivo gene delivery follows a Poisson distribution,." Gene Ther, 43-52), a truncated human TnnT2 promoter (described, for example, in Werfel, et al. (2014), "Rapid and highly efficient inducible cardiac gene knockout in adult mice using AAV-mediated expression of Cre recombinase," Cardiovasc Res, 104(1): p. 15-23), a ventricular myosin light chain 2 (MLC2v) promoter (described, for example, in Phillips et al. (2002), "Vigilant vector: heart-specific promoter in an adeno-associated virus vector for cardioprotection. Hypertension," 39(2 Pt 2): p. 651-5), or an alpha-myosin heavy chain ($\alpha$-MHC) promoter (described, for example, in Gulick, et al. (1991), "Isolation and characterization of the mouse cardiac myosin heavy chain genes," J Biol Chem, 266(14): p. 9180-5). In some embodiments, the promoter is a Cardiac atrial specific promoter, such as an atrial natriuretic factor (ANF) promoter (described, for example, in Ni et al. (2019), "Atrial-Specific Gene Delivery Using an Adeno-Associated Viral Vector," Circ Res, 124(2): p. 256-262).

Regulatable promoter, such as a metallothionein promoter, a heat-shock promoter, and other regulatable promoters, of which many examples are well known in the art are also contemplated. Furthermore, a Cre-loxP inducible system can also be used, as well as a Flp recombinase inducible promoter system, both of which are known in the art.

In some embodiments, the nucleotide sequence encoding the sodium channel polypeptide comprises a sequence encoding a motif. A "motif" as used herein refers to a portion of a polypeptide sequence and includes at least two amino acids. A motif may be 2 to 25, 2 to 20, 2 to 15, or 2 to 10 amino acids in length. In some embodiments, a motif includes 3, 4, 5, 6, or 7 sequential amino acids. For example, the motif may be an export motif or a membrane trafficking motif that directs the sodium channel polypeptide to certain cellular locations such as the cellular membrane. Motifs may include a sequence directing the sodium channel polypeptide for interaction with a particular receptor, antigen, or other polypeptide. Export motifs may include a Golgi export motif. The motif may be an endoplasmic reticulum (ER) export motif. In some embodiments, the motif may be a membrane anchoring signal, such as, for example, a PDZ binding motif or an Ankyrin-G binding motif. In some embodiments, the motif is a tyrosine-based sorting signal, a prenylation motif, a dual palmitoylation signal, a Thy-1 N-terminal GPI-linked signal, or a GPI-attachment signal of lymphocyte-function-associated antigen 3.

In some embodiments, the motif is a trafficking motif such as a Golgi export sequence trafficking signal (for example, KSRITSEGEYIPLDQIDINV; SEQ ID NO: 63), a endoplasmic reticulum (ER) export sequence (for example, FCYENEV; SEQ ID NO: 62), or a potassium channel PDZ-binding motif (for example, SEI; SEQ ID NO: 65). Such motifs are described, for example, in Hofherr et al. (2005), "Selective Golgi export of Kir2.1 controls the stoichiometry of functional Kir2.x channel heteromers," J Cell Sci, 118(Pt 9): p. 1935-43; Leonoudakis et al.

(2004), "Protein trafficking and anchoring complexes revealed by proteomic analysis of inward rectifier potassium channel (Kir2.x)-associated proteins," J Biol Chem, 2004. 279(21): p. 22331-46; and Gong et al. (2014), "Imaging neural spiking in brain tissue using FRET-opsin protein voltage sensors," Nat Commun, Vol. 5, Article 3674. In some embodiments, the motif may be a specific tyrosine-based sorting signal (for example, YSVL; SEQ ID NO: 69) that could mark BacNa$_v$ channels for incorporation into clathrin-coated vesicles at the *trans*-Golgi similar to Kir2.1 and confer specificity on membrane trafficking (see, e.g., Park and Guo, "Adaptor protein complexes and intracellular transport," Biosci Rep, 34(4)).

Membrane targeting can also be achieved by introducing additional posttranslational modification (PTM) for lipid modifications, which often direct proteins to the cell membrane. Several types of lipid modifications can be used including, for example, prenylation (farnesylation and geranylgeranylation), N-myristoylation, S-palmitoylation, and glycosylphosphatidylinositol (GPI) anchoring. See, for example, Haucke et al. (2007), "Lipids and lipid modifications in the regulation of membrane traffic," Curr Opin Cell Biol, 19(4): p. 426-35, for description of such modifications. One of the most widely used prenylation motifs is motif $CX_1X_2X_3$ (SEQ ID NO: 70; $X_1$ = aliphatic, $X_2$ = aliphatic, $X_3$ = any amino acid) at the C-terminus, which will introduce a triplet of PTMs to make protein hydrophobic at their C-termini to facilitate its association with membranes (Hancock et al. (1991), "A CAAX or a CAAL motif and a second signal are sufficient for plasma membrane targeting of ras proteins," EMBO J, 10(13): p. 4033-9). In some embodiments, the motif is a myristoylation sequence, where ddition of the myristoylation sequence (MGXXXSXX; SEQ ID NO: 74) to the N-terminus Gly residue may result in the attachment of myrostic acid to N- terminal Gly residue though an amide linkage (Hayashi and Titani, (2010), "N-myristoylated proteins, key components in intracellular signal transduction systems enabling rapid and flexible cell responses," Proc Jpn Acad Ser B Phys Biol Sci, 86(5): p. 494-508).

In some embodiments, the sequence encoding the motif is selected from the group consisting of SEQ ID NOs: 59-74. The amino acid sequences of SEQ ID NOs: 59-74 are set forth in Table 3.

Table 3. Motifs for use with sodium channel polypeptides

| Motif | Amino acid sequence | SEQ ID NO |
|---|---|---|
| Golgi export motif | RSFVKKDGHCNVQFINV | 59 |
| Endoplasmic Reticulum (ER) export motif | GVKESL | 60 |
| | DLRRSL | 61 |
| | FCYENEV | 62 |
| Membrane trafficking motif | KSRITSEGEYIPLDQIDINV | 63 |
| PDZ binding motif | TDV | 64 |
| | SEI | 65 |
| | SIV | 66 |
| | SLA | 67 |
| Ankyrin-G binding motif | VPIAVAESD | 68 |
| Tyrosine-based sorting signal | YSVL | 69 |
| Prenylation motif | $CX_1X_2X_3$ ( $X_1$ = aliphatic, $X_2$ = aliphatic, $X_3$ = any amino acid) | 70 |
| Dual palmitoylation signal | MLCCMRRTKQ | 71 |
| Thy-1 N-terminal GPI-linked signal | KDNTTLQEFATLAN | 72 |

(continued)

| Motif | Amino acid sequence | SEQ ID NO |
|---|---|---|
| GPI-attachment signal of lymphocyte-function-associated antigen 3 | PSSGHSRYALI | 73 |
| Myristoylation sequence | MGXXXSXX | 74 |

The one or more export motif and/or a membrane trafficking motif may be at the C-terminus, at the N-terminus, at an internal location of the sodium channel polypeptide, or a combination thereof. In some embodiments, the ion channel polypeptide includes a motif at the C-terminal end. In some embodiments, the sodium channel polypeptide further includes a fluorescent tag for detection. Fluorescent tags may include fluorescent polypeptides known in the art such as, for example, GFP, mEGFP, RFP, and YFP. The motif and/or tag may be encoded by a nucleotide sequence in the same reading frame as the nucleotide sequence encoding the sodium channel polypeptide. The motif or tag may be linked to the sodium channel polypeptide via a peptide linker. Peptide linkers may include, for example, $(G)_n$ wherein n is an integer from 1 to 10 (SEQ ID NO: 75); $(GGGGS)_n$ wherein n is an integer from 1 to 10 (SEQ ID NO: 76); $(EAAAK)_n$ wherein n is an integer from 1 to 10 (SEQ ID NO: 77); or $(XP)_n$ wherein n is an integer from 1 to 10 and X is any amino acid (SEQ ID NO: 78); or a combination thereof.

In some instances, functioning of the sodium channel polypeptides may be enhanced by expression of additional polypeptides. For example, a potassium channel polypeptide may be co-expressed with the sodium channel protein to create a steady membrane potential in expressing cells. In some approaches, a connexin protein (also known as gap junction proteins) may be co-expressed to allow the expressing cells to electrically communicate with each other via gap junctions, channels that permits ions and small molecules to move between adjacent cells. Accordingly, in some embodiments, the nucleic acid further comprises a nucleotide sequence encoding at least one of a potassium channel and/or a connexin protein. Potassium channel proteins and connexin proteins used in the present invention may be from any source but are generally human derived, bacterial derived, or engineered derivatives thereof. Potassium channel protein and/or connexin protein sequences may be engineered through synthetic or other suitable means by reference to published sequences such as are available in the literature or in databases such as, e.g., GenBank, PubMed, or the like. Potassium channel proteins and connexin proteins are well known and well-characterized. For example, potassium channel proteins are described in collections such as VKCDB (Li and Gallin (2004), VKCDB: "Voltage-gated potassium channel database", BMC Bioinformatics; 5: 3). Also, see Schak Nielsen, M. et al. (2012), "Gap Junctions," Compr. Physiol., 2(3): 1981-2035.

In some embodiments, the potassium channel protein is an inward-rectifier potassium channel ($K_{ir}$2.1), or variant thereof. In some embodiments, the $K_{ir}$2.1 protein has at least 90% identity to the sequence of SEQ ID NO: 79. In some embodiments, the connexin protein is a connexin 43 (Cx43) protein, or variant thereof. In some embodiments, the connexin protein is a connexin 40 (Cx40) protein, or variant thereof. In some embodiments, the Cx43 protein has at least 90% identity to the sequence SEQ ID NO: 80. In some embodiments, Kir2.1 and/or Cx43 are under control of the same promoter that controls expression of the sodium channel polypeptide. In some embodiments, Kir2.1 and/or Cx43 are operably linked to different promoters.

2.2 Constructs, Vectors, and Viruses

Also provided is a DNA construct comprising a heterologous promoter operably linked to a nucleic acid comprising a nucleotide sequence encoding a sodium channel polypeptide as described herein. Numerous promoters can be used in the constructs described herein. A discussion on suitable promoters is provided above in section 2.1.

The recombinant nucleic acids provided herein can be included in expression cassettes for expression in a cell or an organism of interest. The cassette will include 5' and 3' regulatory sequences operably linked to a nucleic acid provided herein that allows for expression of the sodium channel polypeptide. Regulatory elements include promoters, enhancers, terminator sequences, polyadenylation (polyA) sequences, and the like), mRNA stability sequences (e.g. Woodchuck Hepatitis Virus Posttranscriptional Regulatory Element; WPRE), sequences that allow for internal ribosome entry sites (IRES) of bicistronic mRNA, sequences necessary for episome maintenance, sequences that avoid or inhibit viral recognition by Toll-like or RIG-like receptors (e.g. TLR-7, -8, -9, MDA-5, RIG-I and/or DAI) and/or sequences necessary for transduction into cells. In some instances, the expression cassette includes in the 5' to 3' direction of transcription: a transcriptional and translational initiation region (i.e., a promoter), a polynucleotide of the invention, and a transcriptional and translational termination region (i.e., termination region) functional in the cell or organism of interest.

The expression cassette may comprise none, one or more of any of the regulatory elements described herein. Such an expression cassette is provided with a plurality of restriction sites and/or recombination sites for insertion of the polynucleotides to be under the transcriptional regulation of the regulatory regions. The cassette may additionally contain at least one additional gene or genetic element to be cotransformed into the organism. In some embodiments, the expression cassette includes at least one additional gene encoding a potassium channel protein and/or a connexin protein (see section 2.1 for a discussion on potassium channel proteins and connexin proteins). Where additional genes or elements are included, the components are operably linked. Alternatively, the additional gene(s) or element(s) can be provided on multiple expression cassettes. For example, the multiple expression cassettes might each comprise one of a gene encoding a sodium channel polypeptide, a potassium channel protein, or a connexin protein. The expression cassette may additionally contain a selectable marker gene. The promoters of the invention are capable of directing or driving expression of a coding sequence in a cell. The regulatory regions (i.e., promoters, transcriptional regulatory regions, and translational termination regions) may be endogenous or heterologous to the cell or to each other.

Additional regulatory signals include, but are not limited to, transcriptional initiation start sites, operators, activators, enhancers, other regulatory elements, ribosomal binding sites, an initiation codon, termination signals, and the like. See Sambrook et al. (1992) Molecular Cloning: A Laboratory Manual, ed. Maniatis et al. (Cold Spring Harbor Laboratory Press, Cold Spring Harbor, N.Y.) (hereinafter "Sambrook 11"); Davis et al., eds. (1980) Advanced Bacterial Genetics (Cold Spring Harbor Laboratory Press), Cold Spring Harbor, N.Y., and the references cited therein.

The expression cassette can also comprise a selectable marker gene for the selection of transformed cells. Marker genes include genes conferring antibiotic resistance, such as those conferring hygromycin resistance, ampicillin resistance, gentamicin resistance, neomycin resistance, to name a few. Additional selectable markers are known and any can be used.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

Also provided herein is a vector comprising the nucleic acid comprising a nucleotide sequence encoding a sodium channel polypeptide as described herein. The vector may be a DNA vector or a RNA vector. In some embodiments, the vector is a non-viral vector (e.g., a plasmid or naked DNA) or a viral vector. In some embodiments, the vector is a viral vector. Examples of viral vectors include, but are not limited to, an adeno-associated virus (AAV) vector, a retroviral vector, a lentiviral vector, a herpes simplex viral vector, or an adenoviral vector. It is understood that any of the viral vectors described herein can be packaged into viral particles or virions for administration to the subject.

In some embodiments, the viral vector is an AAV vector. Numerous AAV serotypes are known (see, e.g., Wang et al., "Adeno-associated virus vector as a platform for gene therapy delivery." Nat Rev Drug Discov 18: 358-378 (2019)) including naturally occurring serotypes such as AAV1, AAV2, AAV3, AAV4, AAVS, AAV6, AAV7, AAV8, AAV9, and others. In addition, numerous methods exist and are known to those in the art for engineering novel capsid serotypes (see again, e.g., Wang et al., "Adeno-associated virus vector as a platform for gene therapy delivery." Nat Rev Drug Discov 18: 358-378 (2019). Both naturally occurring and engineered capsid serotypes comprise characteristic tropisms for different species, organs, tissues, cell types, and functions. Each naturally occurring wildtype capsid serotype has a corresponding inverted terminal repeat (ITR) sequence important for viral replication and packaging. In many cases, the genomic ITRs from one capsid serotype can be used to package a genome inside a different capsid serotype. ITRs can also be engineered to improve various characteristics important for therapeutic AAV vectors. See Li, et al., "Engineering adeno-associated virus vectors for gene therapy." Nat Rev Genet 21: 255-272 (2020). Accordingly, AAV vector constructs can be designed so that the AAV ITRs flank the protein coding region. AAV vectors as delivery systems in gene therapy have been well described, e.g. in Dunbar, et al. "Gene therapy comes of age" Science 359:6372 (2018); Penaud-Budloo, et al., "Pharmacology of recombinant Adeno-Associated Virus production" Mol Ther Meth Clin Dev 8: 166-180 (2018); Gonçalves, M.A. "Adeno-associated virus: from defective virus to effective vector." Virol J 2: 43 (2005); Li, et al "Engineering adeno-associated virus vectors for gene therapy." Nat Rev Genet 21: 255-272 (2020); each of which is incorporated by reference for all purposes.

Exemplary AAV vectors useful according to the disclosure include those with genomes existing in either single-stranded (ss) or self-complementary (sc) configurations. AAV sequences that may be used can be derived from the genome of any AAV serotype or may further be engineered. In some embodiments, AAV1, AAV2, AAV3, AAV3, AAV4, AAV5, AAV6, AAV7, AAV8, AAV9, AAV10, AAV11, AAV12, variants of any thereof, or AAVs yet to be discovered or variants thereof may be used as AAV vectors. See, e.g., WO 2005/033321, which is incorporated herein by reference. In some aspects, the AAV vector is a single stranded (ss) AAV vector. In some embodiments, the AAV vector is a self-complementary (sc) vector. In a certain embodiment, the AAV vector is a scAAV9 vector. "Self-complementary AAV" or "scAAV" refers to a vector in which a coding region carried by a AAV nucleic acid sequence has been designed to form an intra-molecular double-stranded DNA template. Upon transduction, rather than waiting for cell-mediated synthesis of the second strand, the two complementary halves of scAAV will associate to form one double-stranded DNA (dsDNA) unit that is ready for immediate replication and transcription. See, e.g., McCarty, et al., "Self-complementary AAV vectors: advances and applications", Mol Ther 16: 1648-1656 (2008). Self-complementary AAVs are described in, e.g., U.S. Pat. Nos. 6,596,535; 7,125,717; and 7,456,683, each of which is incorporated herein by reference in its entirety.

AAV DNA ends comprise an inverted terminal repeat (ITR) characterized by a T-shaped hairpin structure which becomes a 3' hydroxyl group serving as a primer for the initiation of viral DNA replication (Berns K. "Parvovirus replication", Microbiol Rev 54: 316-329 (1990)). The ITRs are the only sequences of viral origin needed to guide genome replication and packaging during vector production. See e.g. Gonçalves, M.A. "Adeno-associated virus: from defective virus to effective vector." Virol J 2: 43 (2005). Accordingly, in some embodiments, the AAV vector comprises an AAV 5' ITRs and an AAV 3' ITS flanking the promoter and the protein coding sequences. The ITR sequences may be from any naturally occurring serotype or they may be engineered. In some embodiments, the ITR is AAV1 ITR, AAV2 ITR, AAV3 ITR, AAV4 ITR, AAV5 ITR, AAV6 ITR, AAV7 ITR, AAV8 ITR, AAV9 ITR, AAV10 ITR, AAV11 ITR, or AAV12 ITR, or variants of any thereof. In one embodiment, ITR sequences may be from AAV1. In one embodiment, ITR sequences may be from AAV6. In one embodiment, ITR sequences may be from AAV9. In some embodiments, full-length AAV ITRs are used. In some embodiments, a shortened version of the AAV ITRs, can be used (see eg., Ling et al., "Enhanced transgene expression from recombinant single-stranded D-sequence-substituted adeno-associated virus vectors in human cell lines in vitro and in murine hepatocytes in vivo. " J Virol 89(2): 952-961 (2015)). In some embodiments, ITRs are selected to generate a single-stranded (ss) AAV vector. In some embodiments, ITRs may be selected to generate a self-complementary AAV vector, such as defined above.

In some embodiments, the viral vector is a lentiviral vector. The lentiviral genome consists of single-stranded RNA that is reverse-transcribed into DNA and then integrated into the host cell genome. Lentiviruses can infect both dividing and non-dividing cells, making them attractive tools for gene therapy. Lentiviruses are also useful for *in vitro* experiments. Methods for making lentiviral vectors are known in the art. See, for example, Tiscornia et al., "Production and purification of lentiviral vectors," Nature Protocols 1: 241-245 (2006); and Keeker et al., "Gene Therapy 2017: Progress and Future Directions,", Clin Transl Sci. 10:242-248 (2017); Higuchi et al., "Direct Injection of Kit Ligand-2 Lentivirus Improves Cardiac Repair and Rescues Mice Post-Myocardial Infarction," Molecular Therapy, 17, no. 2: 262-68 (2009); Zhao et al., "Lentiviral Vectors for Delivery of Genes into Neonatal and Adult Ventricular Cardiac Myocytes in Vitro and in Vivo," Basic Research in Cardiology, 97, no. 5 (2002): 348-58.

In some aspects, the disclosure provides a virus comprising the nucleic acid comprising a nucleotide sequence encoding a sodium channel polypeptide as described herein or the viral vector as described herein. The virus may be a AAV, a lentivirus, or a retrovirus.

In some embodiments, the AAV comprises the AAV vector as described herein and an AAV capsid. The AAV capsid can be of any AAV serotype. For example, the AAV capsid can be an AAV1, AAV2, AAV3, AAV3, AAV4, AAV5, AAV6, AAV7, AAV8, AAV9, AAV10, AAV11, AAV12, or variants thereof. In one embodiment, the AAV capsid is an AAV9 capsid. In some embodiments, the AAV capsid can be from an engineered AAV. In some embodiments, the AAV ITRs may be of the same AAV origin as the capsid employed in the resulting AAV. For example, the AAV vector may contain AAV9 genome ITRs and AAV9 capsid proteins. In other embodiments, the AAV may be pseudotyped, where the ITRs are of one AAV serotype and the capsid proteins are of a different AAV serotype. In some embodiments, the AAV capsid is engineered to be chimeric, comprising sequences from two or more different AAV serotypes.

The AAV described herein may be generated and isolated using methods known in the art. See, e.g., U.S. Pat. Nos. 7,790,449, U.S. Pat. No. 7,588,772, WO 2005/033321, and Zolotukin et al., "Production And Purification Of Serotype 1, 2, And 5 Recombinant Adeno-Associated Viral Vectors." Methods 28:158-167 (2002), incorporated by reference, and Penaud-Budloo et al., 2018; Gonçalves, M.A. "Adeno-associated virus: from defective virus to effective vector." Virol J 2: 43 (2005); Li, et al "Engineering adeno-associated virus vectors for gene therapy." NatRev Genet 21: 255-272 (2020); all incorporated by reference and cited above. For general methods on genetic and recombinant engineering, recombinant engineering, and transfection techniques see e.g., Sambrook et al, Molecular Cloning: A Laboratory Manual, Cold Spring Harbor Press, Cold Spring Harbor, N.Y.; Graham et al., Virol., 52:456 (1973); Davis et al., Basic Methods in Molecular Biology, Elsevier, (1986); and Chu et al., Gene 13:197 (1981). In some embodiments, AAVs may be produced using the triple transfection method (described in detail in U.S. Pat. No. 6,001,650). Cells for producing AAVs are known in the art and include, but are not limited to those capable of baculovirus infection, including insect cells such as High Five, Sf9, Se301, SeIZD2109, SeUCR1, Sf9, Sf900+, Sf21, BTI-TN-5B1-4, MG-1, Tn368, HzAm1, BM-N, Ha2302, Hz2E5 and Ao38, and mammalian cells such as HEK293, HeLa, CHO, NSO, SP2/0, PER.C6, Vero, RD, BHK, HT 1080, A549, Cos-7, ARPE-19 andMRC-5 cells.

In some embodiments, the virus is a lentivirus. Methods for lentivirus production are known in the art. See, for example, Wang and McManus "Lentivirus production," J. Vis. Exp. 32: 1499 (2009). Generally, lentivirus production relies on the transient transfection of suitable cells (e.g., HEK 293T) with a packaging plasmid, an envelope glycoprotein-encoding plasmid and a lentiviral vector. Following transfection, lentiviral particles are produced and released into the culture supernatant of the cells. See e.g., Vigna and Naldini (2000), "Lentiviral vectors: excellent tools for experimental gene transfer and promising candidates for gene therapy,". J Gene Med. 2:308-316; and Segura et al. (2010), "New protocol for lentiviral vector mass production," Methods Mol Biol., 614:39-52. Lentiviruses may also be prepared using second generation lentiviral packaging system (see e.g., Nguyen et al. (2018), "Generation and customization of biosynthetic excitable tissues for electrophysiological studies and cell-based therapies,". Nat Protoc, 13(5):927-945).

Non-viral vectors can also be used to deliver the sodium channel polypeptide(s). Accordingly, in some embodiments, the vector is a non-viral vector. For example, non-viral systems, such as naked DNA formulated as a microparticle, may be used. In some embodiments, delivery may include using virus-like particles (VLPs), cationic liposomes, nanoparticles, cell-derived nanovesicles, direct nucleic acid injection, hydrodynamic injection, use of nucleic acid condensing peptides and non-peptides. In one approach, virus-like particles (VLP's) are used to deliver the sodium channel polypeptide(s). The VLP comprises an engineered version of a viral vector, where nucleic acids are packaged into VLPs through alternative mechanisms (e.g., mRNA recruitment, protein fusions, protein-protein binding). See Itaka and Kataoka, 2009, "Recent development of nonviral gene delivery systems with virus-like structures and mechanisms," Eur J Pharma and Biopharma 71:475-483; and Keeler et al., 2017, "Gene Therapy 2017: Progress and Future Directions" Clin. Transl. Sci. (2017) 10, 242-248, incorporated by reference.

Also provided herein is a nanoparticle comprising the nuleic acid as described herein. Examples of nanoparticles that can be used in the methods and compositions described herein include, gold nanoparticles, silica nanoparticles, polyethyleneglycol/polyethyleneimine particles, or lipid nanooparticles can be used.

## 2.3 Cells

Also provided is a cell comprising any of the nucleic acids or vectors described herein. Populations of any of the cells described herein are also provided. The cell can be a eukaryotic cell, for example, a mammalian cell. In some embodiments, the cell is a human cell. In some embodiments, the cell is a cardiomyocyte (e.g., an atrial or a ventricular cardiomyocyte). In some embodiments, the cell is a stem cell, such as a human induced pluripotent stem cells (hiPSCs). In some embodiments, the cell is a fibroblast (e.g., a human dermal fibroblast). In some instances, the cell is a primary cell. In some instances, the cell is a cell of a cultured cell line.

A cell culture comprising one or more cells described herein is also provided. Methods for the culture and production of cells, including cells of bacterial (e.g., *E. coli* and other bacterial strains), animal (e.g., mammalian), and archebacterial origin are available in the art. See e.g., Sambrook, Ausubel, and Berger (all supra), as well as Freshney (1994) Culture of Animal Cells, a Manual of Basic Technique, 3rd Ed., Wiley-Liss, New York and the references cited therein; Doyle and Griffiths (1997) Mammalian Cell Culture: Essential Techniques John Wiley and Sons, NY; Humason (1979) Animal Tissue Techniques, 4th Ed. W.H. Freeman and Company; and Ricciardelli, et al., (1989) In vitro Cell Dev. Biol. 25:1016-1024.

In some embodiments, the cell is a HEK293T cell, a Chinese hamster ovary (CHO) cell, a COS-7 cell, a HELA cell, an avian cell, a myeloma cell, a Pichia cell, or an insect cell. A number of other suitable cell lines have been developed and include myeloma cell lines, fibroblast cell lines, and a variety of tumor cell lines such as melanoma cell lines.

The vectors containing the nucleic acids of interest can be transferred or introduced into the cell by well-known methods, which vary depending on the type of cellular host. In some embodiments, the cell may comprise a vector comprising a potassium channel protein coding sequence and/or a connexin protein coding sequence. Thus, in some embodiments, the cell expresses at least one of a potassium channel protein and/or a connexin protein. In some instances, the vector comprises the nucleotide sequence encoding the sodium channel polypeptide and a potassium channel protein coding sequence and/or a connexin protein coding sequence. In some instances, the cell may comprise one or more separate vectors comprising a potassium channel protein coding sequence and/or a connexin protein coding sequence. In some embodiments, the potassium channel protein is an inward-rectifier potassium channel ($K_{ir}2.1$). In some embodiments, the $K_{ir}2.1$ protein has at least 90% identity to the sequence of SEQ ID NO: 79. In some embodiments, the connexin protein is a connexin 43 (Cx43) protein. In some embodiments, the Cx43 protein has at least 90% identity to the sequence of SEQ ID NO: 80.

In some instances, the sodium channel polypeptides may be co-expressed with other polypeptides that have beneficial effects on cardiac conditions described herein. In some embodiments, the cell may comprise a vector comprising a sequence encoding an additional polypeptide, such as any one of a dominant-negative TGF-beta Type II Receptor, a dominant-negative KCNH2 potassium channel (KCNH2-G628S), a Calseqsterin (CASQ2), a hyperpolarization-activated cyclic nucleotide-gated channel (HCN2/HCN3/HCN4), an adenylyl cyclase 1 (ADCYI), a TBX18, a Sarcoendoplasmic Reticulum calcium-ATPase 2a (SERCA2A/ATP2a2), a S100 calcium binding protein A1 (S100A1), an adenylyl cyclase type 6 (AC6), an Inhibitor-1 (I-1c), a B-cell lymphoma 2 (Bcl2)-associated athanogene 3 (BAG3), G-Protein-Coupled Receptor Kinase 2 Inhibitor-βARK-ct, or Stromal-derived factor-1 (SDF-1/CXCL12). In some instances, the cell may comprise one or more vectors comprising a sequence encoding one or more of such polypeptides. Thus, in some embodiments, the cell expresses an additional polypeptide, such as one or more of a dominant-negative TGF-beta Type II Receptor, a dominant-negative KCNH2 potassium channel (KCNH2-G628S), a Calseqsterin (CASQ2), a hyperpolarization-activated cyclic nucleotide-gated channel (HCN2/HCN3/HCN4), an adenylyl cyclase 1 (ADCY1), a TBX18, a Sarcoendoplasmic Reticulum calcium-ATPase 2a (SERCA2A/ATP2a2), a S100 calcium binding protein A1 (S100A1), an adenylyl cyclase type 6 (AC6), an Inhibitor-1 (I-1c), a B-cell lymphoma 2 (Bcl2)-associated athanogene 3 (BAG3), G-Protein-Coupled Receptor Kinase 2 Inhibitor-βARK-ct, and/or Stromal-derived factor-1 (SDF-1/CXCL12).

Methods for introducing vectors into cells are known in the art. As used herein, the phrase "introducing" in the context of introducing a nucleic acid into a cell refers to the translocation of the nucleic acid sequence from outside a cell to inside the cell. In some cases, introducing refers to translocation of the nucleic acid from outside the cell to inside the nucleus of the cell. Various methods of such translocation are contemplated, including but not limited to, electroporation, nanoparticle delivery, viral delivery (as discussed above), contact with nanowires or nanotubes, receptor mediated internalization, translocation via cell penetrating peptides, liposome mediated translocation, DEAE dextran, lipofectamine, calcium phosphate or any method now known or identified in the future for introduction of nucleic acids into prokaryotic or eukaryotic cellular hosts. A targeted nuclease system (e.g., an RNA-guided nuclease (for example, a CRISPR/Cas9 system), a transcription activator-like effector nuclease (TALEN), a zinc finger nuclease (ZFN), or a megaTAL (MT) (Li et al. Signal Transduction and Targeted Therapy 5, Article No. 1 (2020)) can also be used to introduce a nucleic acid into a cell.

### 2.4 Pharmaceutical compositions

Also provided herein are pharmaceutical compositions of the nucleic acids, the vectors, the viruses, or the cells described herein. The pharmaceutical compositions described herein are for delivery to subjects in need thereof by any suitable route or a combination of different routes. The pharmaceutical compositions can be delivered to a subject, so as to allow expression of the sodium channel polypeptide in cells of the subject and produce an effective amount of the sodium channel polypeptide that treats a condition in the subject, such as a cardiac condition, as described in more detail in Section 3. In some embodiments, the pharmaceutical composition comprising the nucleic acid, the vector, the virus, or the cell as described herein further comprises a pharmaceutically acceptable excipient or carrier.

The terms "pharmaceutically acceptable carrier" and "pharmaceutically acceptable excipient" are used interchangeably and refer to a substance or compound that aids or facilitates preparation, storage, administration, delivery, effectiveness, absorption by a subject, or any other feature of the composition for its intended use or purpose. Such pharmaceutically acceptable carrier is not biologically or otherwise undesirable and can be included in the compositions of the present invention without causing a significant adverse toxicological effect on the subject or interacting in a deleterious manner with the other components of the pharmaceutical composition.

In some approaches, sterile injectable solutions can be prepared with the vectors in the required amount and an excipient suitable for injection into a human patient. In some embodiments, the pharmaceutically and/or physiologically acceptable excipient is particularly suitable for administration to the cardiac muscle. For example, a suitable carrier may be buffered saline or other buffers, e.g., HEPES, to maintain pH at appropriate physiological levels, stabilizing agents, adjuvants, diluents, or surfactants. In some embodiments, the pharmaceutically acceptable excipient comprises a non-ionic detergent, such as, for example, Pluronic F-68®. For injection, the excipient will typically be a liquid. Exemplary pharmaceutically acceptable excipients include sterile, pyrogen-free water and sterile, pyrogen-free, phosphate buffered saline. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. The preparation of pharmaceutically acceptable carriers, excipients and formulations is described in, e.g., Remington: The Science and Practice of Pharmacy, 22nd edition, Loyd V. Allen et al, editors, Pharmaceutical Press (2012). See also Bennicelli et al., "Reversal of blindness in animal models of leber congenital amaurosis using optimized AAV2-mediated gene transfer," Mol Ther. (2008);16(3):458-65. A variety of known carriers are also provided in U.S. Patent Publication Nos. 7,629,322, and 6,764,845, incorporated herein by reference.

### 2.5 Tissue patch

Aspects of the disclosure further relate to a tissue patch comprising a plurality of cells described herein, where the plurality of cells include a nucleotide sequence encoding a sodium channel polypeptide. In some approaches, the tissue patch is suitable for implantation onto the surface of a cardiac muscle of a subject. In some embodiments, the plurality of cells are cardiomycotes. In some embodiments, the plurality of cells are mammalian cardiomyocytes, e.g., human cardiomyocytes.

The term "tissue patch" as used herein, refers to two or three-dimensional mass of living mammalian tissue produced by *in vitro* culturing and growth. The tissue patch comprises a plurality cells and an extracellular matrix surrounding the cells. In some instances, the tissue patch comprises a plurality of different types of cells.

The term "extracellular matrix" refers to a three-dimensional network of macromolecules (such as proteins) that provides an architectural scaffold for cellular adhesion and migration. For example, the extracellular matrix may include decellularized tissues and organs, collagen-GAG, collagen, fibrin, laminin, fibronectin, basal lamina matrices, proteoglycans, glycoproteins, glycosaminoglycans, PLA, PGA, PLA-PGA co-polymers, poly(anhydrides), poly(hydroxy acids), poly(ortho esters), poly(propylfumerates), poly(caprolactones), polyamides, polyamino acids, polyacetals, biodegradable polycyanoacrylates, biodegradable polyurethanes and polysaccharides, polypyrrole, polyanilines, polythiophene, polystyrene, polyesters, non-biodegradable polyurethanes, polyureas, poly(ethylene vinyl acetate), polypropylene, polymethacrylate, polyethylene, polycarbonates, poly(ethylene oxide), co-polymers of the above, mixtures of the above, or adducts of the above. The extracellular matrix may further include a coating including an agent that promotes cell adhesion, for example, fibronectin, laminin, collagen, integrins, or oligonucleotides that promote cell adhesion. In some embodiments, the extracellular matrix may comprise fibrinogen and/or thrombin, or other polypeptides, or a combination thereof. The matrix can also contain anti-fibrinolytic agents including aminocaproic acid, tranexamic acid, aprotinin, or a combination thereof.

The tissue patch may comprise a plurality of cells described herein and other cell types, such as cardiomyocytes, endothelial cells, endocardial cells, vascular smooth muscle cells, vascular endothelium, fibroblasts, pericytes, immune system cells like macrophages, other stromal cells, or any other cell types. The term also encompasses a three-dimensional mass of living mammalian tissue produced at least in part by growth in vivo.

In some embodiments, the plurality of cells are derived from mammalian fibroblasts (e.g., human dermal fibroblasts) or from stem cells. Stem cells useful for the tissue patch provided herein include, for example, embryonic stem cells, amniotic stem cells, bone marrow stem cells, placenta-derived stem cells, embryonic germ cells, cardiac stem cells, induced pluripotent stem cells, mesenchymal stem cells, and endothelial progenitor cells. The stem cells employed can be autologous or heterologous to the subject being treated. In specific embodiments, the stem cells are autologous stem cells.

Fibroblasts and/or stem cells can be derived from any of a variety of sources. For example, the stem cells may be derived from the subject to be treated. In other embodiments, the stem cells are derived from a donor that is different from the subject to be treated. Donors include, for example, mammals, such as non-primates (e.g., cows, pigs, horses, cats, dogs, rats or rabbits) or primates (e.g., monkeys or humans). In specific embodiments, the donor is a human.

In some approaches, the plurality of cells are cardiomyocytes derived from fibroblasts that have been reprogrammed. In some aspects, reprogramming involves the delivery of microRNA that mediate the reprogramming of fibroblasts into cardiomyocytes. Methods for reprogramming include, for example, those described in, e.g., Li et al. (2016), "Tissue-engineered 3-dimensional (3D) microenvironment enhances the direct reprogramming of fibroblasts into cardiomyocytes by microRNAs," Scientific Reports, 6, 38815; Jayawardena et al. (2012), "MicroRNA-mediated in vitro and in vivo direct reprogramming of cardiac fibroblasts to cardiomyocytes," Circ Res 110, 1465-1473; Jayawardena et al. (2015), "MicroRNA induced cardiac reprogramming in vivo: evidence for mature cardiac myocytes and improved cardiac function," Circ Res 116, 418-424; Jayawardena et al. (2014), "Direct reprogramming of cardiac fibroblasts to cardiomyocytes using microRNAs," Methods Mol Biol 1150, 263-272; Ieda, et al. (2010), "Direct reprogramming of fibroblasts into functional cardiomyocytes by defined factors," Cell 142, 375-386; Qian et al. (2012), "In vivo reprogramming of murine cardiac fibroblasts into induced cardiomyocytes," Nature 485, 593-598; and Song et al. (2012), "Heart repair by reprogramming non-myocytes with cardiac transcription factors," Nature 485, 599-604.

The tissue patch may have any size, shape, and/or geometry suitable for implantation and may be selected based upon the desired placement and/or use. Suitable geometries include, but are not limited to, square, rectangular, circular, oval, triangular, polygonal, irregular, any other suitable geometry for implantation, or a combination thereof. In some aspects, the tissue patch is porous. In some instances, the tissue patch can have a length and/or width from 5 mm to 50 mm, such as 5 mm, 10 mm, 15 mm, 20 mm, 25 mm, 30 mm, 35 mm, 40 mm, 45 mm, 50 mm. In some instances, the tissue patch can have a diameter from 5 mm to 50 mm, such as 5 mm, 10 mm, 15 mm, 20 mm, 25 mm, 30 mm, 35 mm, 40 mm, 45 mm, 50 mm. In some embodiments, the tissue patch has a size of 5 mm × 5 mm, or a size of 10 mm × 10 mm. In another embodiment, the size of the tissue patch is suitable for implantation in larger animals, such as humans and other animals, with dimensions of 15 mm × 15 mm or more. In some embodiments, the tissue patch may have a size of 20 mm × 20 mm, 30 mm × 30 mm, 40 mm × 40 mm, or 50 mm × 50 mm.

The tissue patch may have any thickness suitable for implantation. In some embodiments the tissue patch may have a thickness of 50-1000 $\mu$m, 50-100 $\mu$m, 50-250 $\mu$m, 50-500 $\mu$m, 100-300 $\mu$m, 100-500 $\mu$m, 100-700 $\mu$m, 100-1000 $\mu$m, 250-500 $\mu$m, 250-750 $\mu$m, 250-1000 $\mu$m, 500-750 $\mu$m, 500-1000 $\mu$m, 500-750 $\mu$m, 600-800 $\mu$m, or 750-1000 $\mu$m. In some embodiments, the tissue patch may be comprised of 1-50 cell layers, including, for example, 1-10 layers, 1-25 layers, 10-50 layers, 10-25 layers, 10-40 layers, 25-50 layers, 15-45 layers, and 30-50 layers.

### 3. Methods

### 3.1 Increasing cell conduction

According to various embodiments, expression of a sodium channel polypeptide as described in this disclosure in a cell increases the conduction of the cell. Thus, provided herein are methods of increasing the conduction of a cell comprising introducing the nucleic acid, the vector, the virus, or the pharmaceutical composition described herein into the cell, where introduction of the nucleic acid, the vector, the virus, or the pharmaceutical composition induces the expression of the sodium channel polypeptide and thereby increases conduction of the cell. Such methods are useful for *in vitro* analyses and, as described below, for therapeutic purposes.

The term "conduction", as used herein, refers to the ability of a cell to propagate action potential and spread electrical signal to other cells. As such, conduction can be increased by increasing the number of functional sodium or calcium channels. Conduction may be measured by multiple methods known in the art. For measurements in vitro xtracellular recording arrays including microelectrode array (MEA) systems, optical mapping, and combinations thereof can be used. In patients, conduction is usually assessed non-invasively from standard and augmented electrocardiogram (EKG or ECG) traces. There are markers on ECG traces that are used to assess conduction, including the durations of portions of PR interval as markers of speeds of atrial conduction, AV conduction, and His-Purkinje conduction, and the width of QRS complex as a marker of speed of ventricular conduction. Conduction on the surface of the heart can be mapped by non-invasive electrocardiographic imaging (ECGI), based on multi-electrode recordings from the patient's chest. Conduction can be also mapped inside the heart (in atria or ventricles) using more invasive endocardial mapping catheters and multi-site mapping systems (e.g. NOGA) to identify areas of slow conduction, scar, reentry, and automaticity.

### 3.2 Routes of administration and implantation methods

Aspects of this disclosure include methods of administering the nucleic acid, the vector, the virus, the cell, or the pharmaceutical composition of the present disclosure for treating a cardiac condition in a subject in need of treatment. In some aspects, the cardiac condition is characterized by impaired action potential conduction in the heart of the subject.

Aspects of this disclosure also include methods of administering the nucleic acid, the vector, the virus, the cell, or the pharmaceutical composition of the present disclosure for treating a central nervous system (CNS) disorder, a peripheral nervous system (PNS) disorder, or a skeletal muscle disorder. In some embodiments, the CNS and/or PNS disorder is a condition that is associated or results from a loss of function of a eukaryotic sodium channel.

As used herein, the term "administering", "administration", or "administer" means delivering the pharmaceutical composition as described herein to a target cell or a subject. Administration refers to the act of introducing, injecting or otherwise physically delivering a substance as it exists outside the body (e.g., one or more nucleic acids, vectors, viruses, cells, or pharmaceutical compositions described herein) into a subject. The compositions described herein can be delivered to subjects in need thereof by any suitable route or a combination of different routes. Any suitable route of administration or combination of different routes can be used, including systemic administration (e.g., intravenous, intravascular, or intra-arterial injection), local injection into the heart muscle, local injection into the CNS (e.g., intracranial injection, intracerebral injection, intracerebroventricular, or injection into the Cerebrospinal fluid (CSF) via the cerebral ventricular system, cisterna magna, or intrathecal space), or local injection at other bodily sites (e.g. intraocular, intramuscular, subcutaneous, intradermal, or transdermal injection). In some embodiments, the compositions described herein are administered into the coronary arteries. In some embodiments, the compositions described herein are administered into the coronary sinus.

As used herein the terms "treatment", "treat", or "treating" refers to a clinical intervention made in response to a disease, disorder or physiological condition manifested by a patient or to which a patient may be susceptible. The aim of treatment includes the reduction, alleviation, slowing, or stopping the progression or worsening of a disease, disorder, or condition including reducing or preventing one or more of the effects or symptoms of the disease, disorder, or condition and/or the remission of the disease, disorder or condition, for example, a cardiac condition, in the subject. Thus, in the disclosed methods, treatment can refer to a 10%, 20%, 30%, 40%, 50%, 60%, 70%, 80%, 90%, or 100% reduction in the severity of a cardiac condition. For example, a method for treating a cardiac condition is considered to be a treatment if there is a 10% reduction in one or more symptoms of a cardiac condition in a subject as compared to a control. Thus the reduction can be a 10%, 20%, 30%, 40%, 50%, 60%, 70%, 80%, 90%, 100%, or any percent reduction in between 10% and 100% as compared to native or control levels. It is understood that treatment does not necessarily refer to a cure or complete ablation of the disease or symptoms of the disease.

Administration can be performed by injection, by use of an osmotic pump, by electroporation, or by other means. In some approaches, administration of the compositions of the present disclosure can be performed before, after, or simultaneously with surgical treatment. In some embodiment, the surgical treatment may be a cardiac surgery. Cardiac surgeries include, but are not limited to catheter ablation, pacemaker or implantable cardioverter defibrillator (ICD) implantation, coronary artery bypass graft surgery, left ventricular assist device (LVAD) or right ventricular assist device (RVAD) implantation, heart transplantation surgery, and others.

Aspects of this disclosure further include methods of implanting the tissue patch described herein onto the surface of a cardiac muscle of a subject to treat a cardiac condition. In some embodiments, the tissue patch is implanted on an area of the cardiac muscle having impaired action potential conduction. In some embodiments, multiple tissue patches, e.g., 1-20 tissue patches, are implanted.

The term "implanting," "implantation" or any grammatical variation thereof refers to the act of physically delivering, applying and/or placing a solid material as it exists outside the body (e.g., tissue patch) on an organ, a group of cells, or a tissue of a subject. In some embodiments the organ may be a cardiac muscle. In some embodiments, implanting involves attaching the tissue patch such that the tissue patch is fixated at a certain position on an organ, a group of cells, or a tissue of a subject. In some embodiments, implanting involves a surgical procedure by which the tissue patch is placed on an organ, a group of cells, or a tissue of a subject. In one embodiment, implanting involves a surgical procedure by which the tissue patch is placed onto the surface of a cardiac muscle of a subject.

Identification of an area of the cardiac muscle having impaired action potential conduction may be performed by non-invasive ECG (standard 12 leads) and ECGI measurements, or more invasively via endocardial catheter mapping using standard recording/ablating catheters or 3D catheter mapping systems.

### 3.3 Dosage and effective amounts

Dosage values may depend on the nature of the product and the severity of the condition. It is to be understood that for any particular subject, specific dosage regimens can be adjusted over time and in course of the treatment according to the individual need and the professional judgment of the person administering or supervising the administration of the compositions. Accordingly, dosage ranges set forth herein are exemplary only and are not intended to limit the scope or practice of the claimed composition.

In some embodiments, the amount of pharmaceutical composition administered will be an "effective amount" or a "therapeutically effective amount," *i.e.*, an amount that is effective, at dosages and for periods of time necessary, to achieve a desired result. In some instances, a desired result would include an improvement of a cardiac condition in a subject characterized by impaired action potential conduction in heart of the subject. A desired result can also include an improvement in action potential conduction in a target cell and/or in the heart of a subject, enhanced cardiomyocyte contractility, prolonged survival or a detectable improvement in a symptom associated with cardiac condition that improves patient quality of life. In some instances, a desired result would include improvement of a CNS disorder, PNS disorder, or skeletal muscle disorder, such as improved cognitive function and/or improved muscle function and/or control. Alternatively, if the pharmaceutical composition is used prophylactically, a desired result would include reducing the incidence of one or more symptoms of a cardiac condition, a CNS disorder, PNS disorder, or skeletal muscle disorder.

A therapeutically effective amount of such a composition may vary according to factors such as the disease state, age, sex, weight of the individual, and whether it is used concomitantly with other therapeutic agents. Dosage regimens may be adjusted to provide the optimum response. A suitable dose can also depend on the particular viral vector used, or the ability of the viral vector to elicit a desired response in the individual. A therapeutically effective amount is also one in which any toxic or detrimental effects of the viral vector are outweighed by the therapeutically beneficial effects. Other factors determining a dose can include, e.g., other medical disorders concurrently or previously affecting the subject, the general health of the subject, the genetic disposition of the subject, diet, time of administration, and any other additional therapeutics that are administered to the subject. It should also be understood that a specific dosage and treatment regimen for any particular subject also depends upon the judgment of the treating medical practitioner.

The effective amount of the compositions described herein can be determined by one of ordinary skill in the art. One of skill in the art will appreciate that an effective amount of a composition, for example, comprising an AAV or a lentivirus, can be empirically determined. An effective amount of any of the compositions described herein will vary and can be determined by one of skill in the art through experimentation and/or clinical trials. For example, quantification of genome copies (GC), vector genomes (VG), virus particles (VP), or infectious viral titer may be used as a measure of the dose contained in a formulation or suspension. Any method known in the art can be used to determine the GC, VG, VP or infectious viral titer of the virus compositions of the invention, including as measured by qPCR, digital droplet PCR (ddPCR), UV spectrophotometry, ELISA, next-generation sequencing, or fluorimetry as described in, e.g. in Dobkin et al., "Accurate Quantification and Characterization of Adeno-Associated Viral Vectors." Front Microbiol 10: 1570-1583 (2019); Lock et al., "Absolute determination of single-stranded and self-complementary adeno-associated viral vector genome titers by droplet digital PCR" Hum Gene TherMethods 25: 115-125 (2014); Sommer, et al., "Quantification of adeno-associated virus particles and empty capsids by optical density measurement." Mol Ther 7: 122-128 (2003); Grimm, et al. "Titration of AAV-2 particles via a novel capsid ELISA: packaging of genomes can limit production of recombinant AAV-2." Gene Ther 6: 1322-1330 (1999); Maynard et al., "Fast-Seq: A Simple Method for Rapid and Inexpensive Validation of Packaged Single-Stranded Adeno-Associated Viral Genomes in Academic Settings." Hum Gene Ther 30(6): 195-205 (2019); Piedra, et al., "Development of a rapid, robust, and universal picogreen-based method to titer adeno-associated vectors." Hum Gene Ther Methods 26: 35-42 (2015); which are incorporated herein by reference. For intravenous injection, an exemplary human dosage range in vector particles (vp) may be between 5x10e13 - 10x10e14 vp per kilogram bodyweight (vp/kg) in a volume of 1-100,000 µl. In one embodiment, an exemplary human dose for intramuscular (cardiac muscle injection) or intracoronary delivery may be 1x10e14 - 5x10e14 vp per injection into the heart in a volume of 1-1000 µl.

In one approach, the composition is administered in a single dosage selected from those above listed. In another embodiment, the method involves administering the compositions in two or more dosages (e.g., split dosages). In another embodiment, multiple injections are made at different locations. In another embodiment, a second administration of the composition is performed at a later time point. Such time point may be weeks, months or years following the first administration. In some embodiments, multiple treatments may be required in any given subject over a lifetime. Such additional administration is, in one embodiment, performed with a viral vector that is different than the viral vector from the first or previous administration. In some embodiments, such additional administration is performed with an AAV having a different capsid serotype than the AAV from the first or previous administration. In another embodiment, such additional administration is performed with an AAV having the same capsid serotype as the AAV from the first or previous administration.

In some embodiments, expression of the sodium channel is regulated by an inducible promoter (see discussion on promoters in section 2.1). Accordingly, following administration of a composition described herein to a subject, the subject also may be administered an agent that acts as an inducer of expression of the sodium channel polypeptide in the cells of the subject. Exemplary agents that may be used as inducers include tetracycline, mifepristone, or ecdysone. See, e.g., Rivera at al. (2005), "Long-term pharmacologically regulated expression of erythropoietin in primates following AAV-mediated gene transfer," Blood, 105 (4): 1424-1430.

### 3.4 Combination therapies

In some approaches, the compositions of the present disclosure are used in combination with one or more additional agents and/or therapies, including any known, or as yet unknown, agent or therapy that helps slowing progression of, reversing, or ameliorating the symptoms of a cardiac condition. The one or more additional agents and/or therapies may be administered and/or performed before, concurrent with, or after administration of the compositions described herein. The combined administration includes co-administration, using separate formulations or a single pharmaceutical formulation. In some embodiments, the compositions described herein are used in combination with one or more agents, including, but not limited to antiarrhythmic agents (e.g., amiodarone, flecainide, ibutilide, procainamide, propafenone), anticoagulants, angiotensin converting enzyme (ACE) inhibitors, angiotensin receptor blockers, platelet agglutination inhibitors, HMG-CoA reductase inhibitors, beta-blockers, phosphodiesterase inhibitors, diuretics, mineralocorticoid receptor agonists, inotropic drugs. In some embodiments, the compositions described herein are used in combination with one or more surgical treatments, such as catheter ablation (e.g., radiofrequency ablation, or cryoablation), pacemaker or implantable cardioverter defibrillator (ICD) implantation, coronary artery bypass graft surgery, left ventricular assist device (LVAD) or right ventricular assist device (RVAD) implantation, heart transplantation surgery, and others.

In some approaches, the compositions of the present disclosure are used in combination with one or more additional agents and/or therapies, including any known, or as yet unknown, agent or therapy which helps slowing progression of, reversing, or ameliorating the symptoms of a CNS or PNS disorder, such as Dravet Sydrome, Severe idoppathic generalized epilepsy of infancy, Benign familial neonatal-infantile seizures, Autism spectrum disorders, epilepsy, Brugada Syndrome, Brugada Syndrome Type 1, Ataxia, Congenital Insensitivity to Pain, or Anosmia. In some embodiments, the compositions of the present disclosure are used in combination with one or more additional agents and/or therapies, including any known, or as yet unknown, agent or therapy which helps slowing progression of, reversing, or ameliorating the symptoms of a skeletal muscle disorder, such as Duchenne Muscular Dystrophy (DMD), Becker's Muscular Dystrophy (BMD), Congenital Muscular Dystrophy (CMD), and Limb-Girdle Muscular Dystrophy, Myotonic dystrophy type 1 and type 2, and Emery-Dreifuss muscular dystrophy.

### 3.5 Patients and treatable conditions

Patients or subjects who are candidates for treatment with the compositions described herein include those experiencing or having experienced one or more signs, symptoms, or other indicators of a disease or disorder described below. The term "subject" or "patient" refers to an animal (particularly a mammal) and other organisms that receive either prophylactic or therapeutic treatment. For example, a subject can be a mammal such as a primate, and, particularly, a human. Non-human primates are subjects as well. The term subject includes domesticated animals, such as cats, dogs, etc., livestock (for example, cattle, horses, pigs, sheep, goats, etc.) and laboratory animals (for example, ferret, chinchilla, mouse, rabbit, rat, gerbil, guinea pig, etc.). Thus, veterinary uses and medical formulations are contemplated herein.

In some approaches, patients are selected for treatment based on signs, symptoms, clinical phenotypes, and/or biomarkers. In some embodiments, they may be assessed via a clinical exam, including but not limited to heart-monitoring tests (such as electrocardiogram (ECG), echocardiogram, or implantable loop recorder, each with or without a stress test), imaging (e.g., coronary angiography or cardiac computed tomography (CT)), biopsy, or bloodwork.

In one aspect, administration of the compositions disclosed herein at a very early stage disease progression may provide superior therapeutic benefit. For example, treatment may be performed prior to the appearance of signs or symptoms of the disease or when strong risk factors or only early stage signs or symptoms of the disease are observed in the subject. Thus, provided herein are methods and compositions for preventing advanced development of the disease. For example, methods and compositions described herein may be useful for reducing the incidence, progression, and/or severity of a cardiac condition. In some approaches, the patient has no symptoms of a cardiac condition. In some approaches, patients are assessed by genotyping to determine their individual genetics (e.g., by assessing the presence of risk alleles associated with one or more diseases described below) and associated risk of disease. Accordingly, in some approaches, at the time of first administration of the composition, the patient does not exhibit any of the clinical phenotypes of the disease, such as a cardiac condition.

The compositions and methods as described in this disclosure find particular use for treatment of patients or subjects with, or at risk of developing, a cardiac condition characterized by impaired action potential conduction in the heart of the subject. For example, the cardiac condition may be one or more of cardiac arrhythmia, atrial fibrillation, ventricular fibrillation, atrioventricular block, ventricular tachycardia, heart failure, damage from myocardial infarction, damage from stroke, brugada syndrome, left bundle branch block, or chronic ischemia.

In some embodiments, patients receiving therapy with the compositions described in the present disclosure may include those with, or at risk of developing, a central nervous system (CNS), a peripheral nervous system (PNS), or a skeletal muscle disorder. In some embodiments, the CNS and/or PNS disorder is a condition that is associated or results from a loss of function of a eukaryotic sodium channel. Exemplary conditions include Dravet Sydrome, Severe idoppathic generalized epilepsy of infancy, Benign familial neonatal-infantile seizures, Autism spectrum disorders, epilepsy, Brugada Syndrome, Brugada Syndrome Type 1, Ataxia, Congenital Insensitivity to Pain, or Anosmia. Exemplary skeletal muscle disorders include, but are not limited to Duchenne Muscular Dystrophy (DMD), Becker's Muscular Dystrophy (BMD), Congenital Muscular Dystrophy (CMD), and Limb-Girdle Muscular Dystrophy, Myotonic dystrophy type 1 and type 2, and Emery-Dreifuss muscular dystrophy.

In some embodiments, conditions may include those associated with or resulting from a mutation listed in Table 4, or the human mutation corresponding thereto. Shown in Table 4 are transgenic mice with mutations that result in loss-of-function of a sodium channel in the heart. The mutations currently known to be relevant for human disease are indicated with an asterisk (*).

Table 4. Mutations associated with loss-of-function in sodium channels

| Protein | Mouse transgenic line | Mechanism |
|---|---|---|
| Na$_v$1.5 $\alpha$-subunit | *Scn5a+/- | Reduced Na$_v$1.5 mRNA and protein expression Negative shift in Na$_v$1.5 inactivation curve (Papadatos. G. A., et al. PNAS 2002. 9, 6210-6215) |
| Na$_v$1.5 $\alpha$-subunit | Scn5a $\Delta$SIV knock-in (homozygous) | Reduced Na, 1.5 anchoring on lateral membranes due to loss of binding to PDZ domain of $\alpha$1-syntrophin (Shy. D., et al. Circulation 2014, 130, 147-60) |
| Na$_v\beta$3 | *Scn3b-/- | Reduced membrane expression or percentage of functional Na$_v$1.5 channels: Negative shift in Na$_v$1.5 inactivation curve (Hakim, P., et al. Prog. Biophys. Mol. Biol. 2008. 98, 251-266) |
| Ankyrin-G | *Ank3$^{flox/flox}$ $\times$ $\alpha$MHC-cre | Decreased targeting of Na$_v$ 1.5 at intercalated disc Membranes (Makara. M. A., et al. Circ. Res. 2014. 115, 929-938) |
| Plakophilin-2 | *Pkp2+/- | Desmosomal deficiency leading to reduced Na$_v$1.5 localization at intercalated discs; Negative shift in Na$_v$ 1.5 inactivation curve Slowed Na$_v$1.5 recovery from inactivation (Cerrone, M., et al. Cardiovasc. Res. 2012, 95. 460-468; Grossmann. K. S., et al. J. Cell Biol. 2004, 167, 149-160) |
| Desmoglein-2 | $\alpha$MHC-Dsg2-N271S | Decreased Na$_v$1.5 expression at intercalated disc Membranes (Rizzo, S., et al. Cardiovasc. Res. 2012. 95, 409-418; Pilichou, K., et al. J. Exp. Med. 2009, 206, 1787-1802) |

(continued)

| Protein | Mouse transgenic line | Mechanism |
|---|---|---|
| GPD1-L | *GPD1L$^{flox/flox}$ $\times$ $\alpha$MHC-cre | Decreased Na$_v$1.5 membrane trafficking (Mehdi. H.. et al. Circulation 2014, 130, A17871-A17871) |
| Ryanodine receptor 2 | Ry-R2-P2329S/ P2328S | Elevated cytosolic Ca$^{2+}$, leading to reduced Na$_v$1.5 protein and membrane expression Positive shift in Na$_v$1.5 activation curve Enhanced I$_{Na}$ slow inactivation (Goddard. C. A., et al. Acta Physiol. (Oxf). 2008, 194, 123-140; King, J. H.. et al. Cardiovasc. Res. 2013, 99. 751-759) |
| PDK1 | PDK1$^{flox/flox}$ $\times$ $\alpha$MHC-Cre | Increased activity of Foxo1 transcription factor, leading to decreased expression of Na$_v$1.5 (Han. Z., et al. PLoS One 2015, 10. e0122436) |

### 3.6 Producing a tissue patch

Aspects of the disclosure further relate to a method of producing a tissue patch comprising (i) seeding a plurality of cells described herein on a solid support, thereby forming a cell-seeded construct, and (ii) culturing the cell-seeded construct in a culture medium for a period of time, thereby producing the tissue patch. The cell-seeded construct is typically cultured until at least 30% of 3-dimensional confluence is achieved. In some instances, the electrophysiological properties of the developing tissue patch can be monitored as described below to assess maturation of the tissue patch.

The term "solid support" as used herein refers to any solid or stationary material having a surface used to form the plurality of cells into a three-dimensional shape. Solid supports include, for example and without limitation, polymer, insoluble polymer, plastic, glass, biocompatible polymer or matrix, microparticle, nanoparticle, monolayers, bilayers, commercial membranes, resins, matrices, fibers, polymers, gold, silicon, and organic and inorganic metals.

In some embodiments, the solid support is a mold. A The term "mold" refers to a cavity or surface comprising a polymeric base material having an outer surface and a shape configured to a produce tissue patch in a configuration suitable for implantation onto the surface of a heart. In one embodiment, the base material of the mold is polydimethylsiloxane. In another embodiment, the outer surface of the mold comprises a polydimethylsiloxane coating. In a further embodiment, the mold comprises at least one post arranged and disposed to form a pore in the tissue patch as the cells grow around the post. Additionally or alternatively, the mold comprises a plurality of posts arranged and disposed to form a mesh configuration in the tissue patch. The posts may be in any suitable geometry, including, but not limited to, square, rectangular, circular, oval, triangular, polygonal, irregular, any other suitable geometry for forming the desired pore(s), or a combination thereof. In other embodiments, the mold may be devoid of posts to form a plain sheet of a tissue patch.

In some approaches, the method includes mixing the cells with a substrate prior to seeding the cells on a solid support (e.g., a mold). A substrate may be used to facilitate the creation of extracellular matrix and new tissue in three dimensions. More specifically, substrates serve as templates to which seeded cells can attach or adhere and provide the cells with the biomechanical support they initially need to grow, differentiate, and organize into three-dimensional structures. The substrate should be compatible with the types of cells that are used in the preparation of the tissue patch and should exhibit a suitable surface chemistry for cell attachment, proliferation and/or differentiation.

The selection of a substrate for use in the preparative methods of the invention will depend on the intended purpose of the tissue patch. Different properties of the substrate may be considered. These properties include, but are not limited to, biocompatibility, biodegradability, tensile strength, flexibility, and elasticity. For example, when the tissue patch is intended to be used as a medical implant, the material should be biologically compatible for implantation into a subject. Furthermore, the substrate may display mechanical properties that match those of the native tissues at the site of implantation. Additionally or alternatively, it may be desirable to control the degradation and resorption rate of the support material to match cell/tissue growth in vitro and/or in vivo.

In some embodiments, the substrate may be in the form of a gel or a foam. Suitable substrates may include a naturally-occurring polymer, a synthetic polymer, or a combination of natural and/or synthetic polymers. Naturally-occurring polymers include polysaccharides and proteins. Exemplary polysaccharides include starches, dextrans, celluloses, hyaluronic acid and its derivatives; exemplary proteins include collagen and gelatin. Polysaccharides such as starches, dextrans, and celluloses may be unmodified or may be modified physically or chemically to affect one or more of their properties such as their characteristics in the hydrated state, their solubility, or their half-life in vivo.

In certain embodiments, the substrate includes a biocompatible, degradable polymer. Such polymers can be broken down by cellular action and/or by action of non-living body fluid components. A variety of biocompatible, degradable polymers may be used and include, but are not limited to, polyanhydrides, polyorthoesters, polyphosphazenes, polycaprolactones, polyamides, polyurethanes, polyesteramides, polydioxanones, polyacetals, polyketals, polycarbonates, polyorthocarbonates, polyhydroxybutyrates, polyhydroxyvalerates, polyalkylene oxalates, polyalkylene succinates, poly(malic acid), poly(amino acids), poly(methyl vinyl ether), poly(maleic anhydride), chitin, chitosan, and copolymers, terpolymers, or higher poly-monomer polymers thereof, or combinations or mixtures thereof. In other embodiments, the polymer includes polyhydroxy acids such as polylactic acid (PLA), polyglycolic acid (PGA), their copolymers poly(lactic-co-glycolic acid) (PLAGA), and mixtures of any of these. These polymers are among the synthetic polymers approved for human clinical use as surgical suture materials and in controlled release devices. Other suitable substrates may be used, for example those described in "Principles of Tissue Engineering, 2nd Edition", R. Lanza, R. Langer, and J. Vacanti (Eds.), Academic Press, 2000; "Methods of Tissue Engineering", A. Atala and R. Lanza (Eds.), Academic Press, 2001; "Animal Cell Culture", Masters (Ed.), Oxford University Press: New York, 2000; U.S. Pat. No. 4,963,489 and related U.S. patents).

In some embodiments, the substrate comprises MATRIGEL™, growth factor-reduced Matrigel. In some embodiments, the substrate may further comprise one or more of laminin, fibrin, fibronectin, thrombin, proteoglycans, glycoproteins, glycosaminoglycans, or growth factors. In some embodiments, Thrombin and fibrinogen, may be derived from the subject (e.g., from biological sample such as blood).

In some embodiments, the substrate is provided onto the mold before seeding the plurality of cells onto the mold, thereby forming a support matrix in which the cells are incorporated. See e.g., U.S. Pat. Nos. 10,106,776.

The method of producing the tissue patch comprises culturing the cell-seeded construct in a culture medium. The cultivation of the cell-seeded construct may be carried out by any suitable method and any suitable culture medium that leads to the formation of a three-dimensional tissue patch exhibiting properties suitable for implantation onto the surface of the heart of a subject. Generally, culture media contain essential nutrients, trace elements, vitamins, lipids, electrolytes, and sources of energy. Other additives include serum from fetal, new born or adult cows, growth factors, cytokines, and functional modulators. The choice of the culture medium and of its components, which affect the proliferation, growth, and function of cells and alter cell phenotype in culture, will depend on the mammalian cells used in the preparative methods. Media for the culture of mammalian cardiac cells are known in the art (see, for example, S. N. Mohamed et al., In Vitro Cell and Develop. Biol. 1983. 19: 471-478; P. Libby, J. Mol. Cell. Cardiol. 1984. 16: 803-811; D. L. Freerksen et al., J. Cell. Physiol., 1984, 120: 126-134; G. Kessler-Icekson et al., Exp. Cell Res. 1984. 155: 113-120; J. S. Karliner et al., Biochem. Biophys. Res. Comm. 1985. 128: 376-382; T. Suzuki et al., FEBS Letters, 1990, 268: 149-151; T. Suzuki et al., J. Cardiov. Pharmacol. 1991, 17: S182-S186; T. Suzuki et al., J. Mol. Cell. Cardiol. 1997, 29: 2087-2093). Different factors and agents may be added to the culture medium. In certain embodiments, the in vitro cultivation of the cell-seeded construct is carried out under conditions selected to promote deposition of extracellular matrix components. The in vitro cultivation of the cell-seeded construct may also be carried out under conditions that promote cell proliferation and/or cell differentiation.

In some embodiments, culturing methods further include the step of treating the cell-seeded construct with at least one biologically active agent. The biologically active agent may include one or more of growth factors, adhesion factors, soluble extracellular matrix proteins, antibiotics, agents that enhance vascularization, agents that enhance cell differentiation, agents that enhance tissue differentiation, agents that inhibit fibrosis, agents that inhibit tumorigenesis, agents that enhance cell proliferation, agents that inhibit cell proliferation, agents that inhibit scaffold degradation, agents that enhance scaffold degradation, agents that enhance histocompatibility, and agents that enhance hemocompatibility.

The cell-seeded construct may be cultured for any suitable duration until a tissue patch is formed exhibiting properties suitable for implantation. In some embodiments, the cell-seed construct may be cultured for 10-20 days. In some embodiments, the cell-seeded construct may be cultured for 12-14 days. In some embodiments, the cell-seeded construct is cultured for 7, 8, 9, 10, 11, 12, 13, or 14 days. The progress of the formation of a tissue patch may be assessed using different methods. See e.g., Zhang et al. (2013), "Tissue-engineered cardiac patch for advanced functional maturation of human ESC-derived cardiomyocytes," Biomaterials, 34(23):5813-20; Bian et al. (2014), "Robust T-tubulation and maturation of cardiomyocytes using tissue-engineered epicardial mimetics," Biomaterials, 35(12):3819-28; Bian et al., (2014), "Controlling the structural and functional anisotropy of engineered cardiac tissues," Biofabrication, 6(2):024109; Hinds et al. (2011), "The role of extracellular matrix composition in structure and function of bioengineered skeletal muscle,"Biomaterials 32, 3575-3583; Liau et al. (2011), "Pluripotent stem cell-derived cardiac tissue patch with advanced structure and function," Biomaterials 32, 9180-9187; Bian et al. (2012), "Local tissue geometry determines contractile force generation of engineered muscle networks," Tissue Eng. Part A 18, 957-967). For example, the spontaneous or stimulated contractile activity of the cell-seeded construct may be determined non-invasively. In some embodiments, the structural and/or biomechanical properties of the cell-seeded construct may be assessed. Properties such as strength, elasticity, conductivity, tissue organization, cellular organization, cell viability, cell morphology, metabolic activity, cell cycle propagation, ultrastructural features, electrical signal propagation, gene expression and protein expression may be studied on small pieces of the cell-seeded construct. Alternatively or additionally, samples of the culture medium may be analyzed for the presence of metabolites and waste products. In some embodiments, the cell-seeded construct may be stimulated electrically, chemically, or mechanically, or a combination thereof.

The choice of the specific cultivation conditions will depend on the nature of the tissue patch desired as well as on the intended purpose of the tissue patch. In certain embodiments, after preparation, the tissue patch is stored before being used. In other embodiments, the tissue patch is used immediately after preparation.

## 4. Exemplary embodiments

Exemplary embodiments of the invention include:

1. A nucleic acid comprising a nucleotide sequence encoding a sodium channel polypeptide comprising an amino acid sequence having at least 90% identity to the sequence of any one of SEQ ID NOs: 1-29, wherein the nucleotide sequence is operatively linked to a heterologous promoter.

2. A nucleotide sequence encoding a sodium channel polypeptide comprising an amino acid sequence having at least 90% identity to the sequence of any one of SEQ ID NOs: 1-29, wherein the nucleotide sequence is not a naturally occurring sequence encoding the sodium channel polypeptide.

3. The nucleic acid of embodiment 1 or embodiment 2, wherein the nucleotide sequence is codon optimized for expression of the sodium channel polypeptide in human cells.

4. The nucleic acid of any one of embodiments 1 to 3, wherein the nucleotide sequence is selected from the group consisting of SEQ ID NOs: 30-58.

5. The nucleic acid of any one of embodiments 1 to 4, wherein the nucleotide sequence comprises at least one of a sequence encoding a motif , wherein the sequence encoding a motif is selected from the group consisting of SEQ ID NOs: 59-74.

6. The nucleic acid of any one of embodiments 1 to 5, wherein the nucleic acid comprises a sequence encoding at least one of a potassium channel and/or a connexin protein.

7. A vector comprising the nucleic acid of any one of embodiments 1 to 6.

8. The vector of embodiment 7, wherein the vector is a viral vector.

9. The vector of embodiment 8, wherein the viral vector is a an adeno-associated viral (AAV) vector, a lentiviral vector, or a retroviral vector.

10. A virus comprising the nucleic acid of any one of embodiments 1 to 6 or the vector of any one of embodiments 7 to 9.

11 . The virus of embodiment 10, wherein the virus is an AAV, a lentivirus, or a retrovirus.

12. A cell comprising the nucleic acid of embodiment 1 to 6, the vector of any one of embodiments 7 to 9 or the virus of embodiment 10 or 11.

13. The cell of embodiment 12, wherein the cell is a mammalian cell.

14. A pharmaceutical composition comprising the nucleic acid of embodiment 1 to 6, the vector of any one of embodiments 7 to 9, the virus of embodiment 10 or 11, or the cell of embodiment 12 or 13, and a pharmaceutically acceptable excipient.

15. A tissue patch comprising a plurality of cells of embodiment 12 or 13.

16. A method of treating a cardiac condition characterized by impaired action potential conduction in the heart, comprising administering to a subject in need thereof a therapeutically effective amount of the nucleic acid of embodiment 1 to 6, the vector of any one of embodiments 7 to 9, the virus of embodiment 10 or 11, the cell of embodiment 12 or 13, or the pharmaceutical composition of embodiment 14.

17. A method of treating a cardiac condition characterized by impaired action potential conduction in the heart, comprising implanting in a subject in need thereof the tissue patch of embodiment 15 onto the surface of a cardiac muscle of the subject, wherein the tissue patch is implanted on an area of the cardiac muscle having impaired action potential conduction.

18. The method of embodiment 16 or embodiment 17, wherein the cardiac condition is one or more of cardiac arrhythmia, atrial fibrillation, ventricular fibrillation, atrioventricular block, ventricular tachycardia, heart failure, damage from myocardial infarction, damage from stroke, brugada syndrome, left bundle branch block, or chronic ischemia.

19. A method of treating a central nervous system (CNS) disorder, a peripheral nervous system (PNS) disorder, or a skeletal muscle disorder, comprising administering to a subject in need thereof a therapeutically effective amount of the nucleic acid of embodiment 1 to 6, the vector of any one of embodiments 7 to 9, the virus of embodiment 10 or 11, the cell of embodiment 12 or 13, or the pharmaceutical composition of embodiment 14.

20. The method of any one of embodiment 16 to embodiment 19, wherein the subject is a human.

21. A method of increasing the conduction of a cell comprising introducing the nucleic acid of embodiment 1 to 6, the vector of any one of embodiments 7 to 9, or the virus of embodiment 10 or 11, wherein introduction of the nucleic acid, the vector, or the virus induces the expression of the sodium channel polypeptide, thereby increasing conduction of the cell.

22. The method of embodiment 21, wherein the cell is a mammalian cell.

23. The method of embodiment 21 or embodiment 22, wherein the cell is a cardiomyocyte.

24. A method of producing a tissue patch comprising:

(i) seeding a plurality of cells of any one of embodiments 12 to 13 on a solid support, thereby forming a cell-seeded construct; and

(ii) culturing the cell-seeded construct in a culture medium for a period of time, thereby producing the tissue patch.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

## 5. Examples

### 5.1 Example 1. Identification and validation of BacNa$_v$ orthologs

_Plasmid construction and lentivirus production._ Fifty novel **BacNa$_v$** orthologs were identified from Uniprot bacteria database and their corresponding cDNAs were codon optimized, synthesized (IDT) and subcloned into pRRL-CMV lentiviral transfer plasmids where they were linked with GFP via the T2A peptide (pRRL-CMV-BacNav-GFP. High-titer lentiviruses were prepared using second generation lentiviral packaging system as described previously (Nguyen et al. (2018), "Generation and customization of biosynthetic excitable tissues for electrophysiological studies and cell-based therapies," Nat Protoc, 13(5): p. 927-945). To determine functional titer of lentiviruses expressing fluorescence reporter, 293T cells were transduced with serial dilutions of concentrated lentiviral stock and the percentage of transduced cells was determined via flow cytometry 72 hours post-transduction. Functional titer in transduction units per mL (TU/mL) was estimated from dilutions that yielded 5 -30% transduction efficiency, by dividing the total number of transduced cells by the volume of virus added in mL.

_Optical mapping of action potential propagation._ For preliminary screening, HEK293 cells stably expressing Kir2.1 and connexin-43 (293KC cells) were transduced with lentiviruses encoding BacNa$_v$ candidates with multiplicity of infection (MOI) of 1. Confluent cell monolayers were optically mapped with a 20-mm diameter hexagonal array of 504 optical fibers (Redshirt Imaging), as previously described (Klinger and Bursac (2008), "Cardiac cell therapy in vitro: reproducible assays for comparing the efficacy of different donor cells," IEEE Eng Med Biol Mag, 27(1): p. 72-80; Kirkton and Bursac (2011), "Engineering biosynthetic excitable tissues from unexcitable cells for electrophysiological and cell therapy studie," Nat Commun, 2: p. 300). Specifically, monolayers were stained with 10 $\mu$M Di-4-ANEPPS for 5 min at room temperature before being transferred to a temperature-controlled (37°C) recording chamber filled with Tyrode's solution. Illumination via a solid-state excitation light source (Lumencor, SOLA SM) was passed through a $520\pm30$ nm bandpass filter to excite the dye and emitted red fluorescence signals ($\lambda > 590$ nm) were collected by the optical fiber array, converted to voltage signals by photodiodes, and recorded at a 2.4-kHz sampling rate with a 750-$\mu$m spatial resolution. Action potential propagation was initiated by 10-ms, 1.2 x threshold, 1-Hz stimuli from a bipolar point electrode connected to a Grass Stimulator (Grass Technologies). Maximum capture rate (MCR) was determined as the highest pacing rate at which tissue responded in 1:1 fashion. Generation of isochrone maps and calculation of CV and APD80 were performed for 1 Hz pacing using custom MATLAB software, as previously described (Bursac et al. (2004), "Multiarm spirals in a two-dimensional cardiac substrate," Proc Natl Acad Sci USA, 2004. 101(43): p. 15530-4; Badie and Bursac, "Novel micropatterned cardiac cell cultures with realistic ventricular microstructure,". Biophys J, 96(9): p. 3873-85).

Whole-cell patch clamp recordings. Transduced 293KC cells were dissociated as single cells and plated onto Aclar coverslip and left to attach for five hours in 37°C incubator. Coverslip was then transferred to a glass-bottom patch clamp chamber perfused with bath solution. Patch pipettes were fabricated with tip resistances of 1-2 M$\Omega$ when filled with pipette solution. Whole-cell patch clamp recordings were acquired at room temperature 25°C using the Multiclamp 700B amplifier (Axon Instruments), filtered with a 10-kHz Bessel filter, digitized at 40 kHz, and analyzed using WinWCP software (John Dempster, University of Strathclyde). To measure activation properties of voltage-gated sodium channels, membrane voltage was stepped from a holding potential of -80 mV to varying 500-ms test potentials (-50 to 60 mV, increments of 10 mV). Inactivation of voltage-gated sodium channels was derived from peak currents measured at 0 mV after varying 3-s prepulse potentials (-160 to -30 mV, increments of 10 mV).

Results. Of the fifty BacNav orthologs, twenty-nine channels showed sodium current, as confirmed with patch clamp recordings. These functional sodium channels exhibited a wide range of gating kinetics indicated by their lowest activation and inactivation time constant (Fig. 1A and Fig. 1B) and voltage dependency (Fig. 1C). The extreme low values of $V_{\frac{1}{2}}$ in Fig. 1C (<-25mV) suggests that most of the channels have overdepolarized activation curve and are active outside of physiological voltage range. The result is confirmed by the preliminary optical mapping studies, which showed that 7 channels enabled successful action potential propagation when co-expressed with Kir2.1 and Cx43 in HEK293 cells (Fig. 2A, Fig. 2B, and FIG. 2C). The two fastest-conducting orthologs (BacNav 10 and BacNav 19) were selected and monoclonal selection and seeding condition optimization was performed for further mapping studies and observed that both BacNav 10 (CV=$20\pm1.3$ cm/s, APD=$105.5\pm1.9$ ms, MCR=$3.25\pm0.25$ Hz) and BacNav19 (CV=$26.8\pm0.82$ cm/s, APD=$47.3\pm0.78$ ms, MCR=$13.5\pm0.29$ Hz) led to faster conduction speed (up to 1-fold) compared to our previously described best therapeutic candidate NavSheP D60A (CV=$13.5\pm0.9$ cm/s, APD=$63.3\pm2.2$ ms, MCR=$4.5\pm0.65$ Hz) (Fig. 3).

## 5.2 Example 2. BacNav mutagenesis and characterization - Kinetics

Mutagenesis of the newly identified BacNav orthologs described herein will be performed to create channels with faster activation and inactivation kinetics than currently available, thus improving the mutated BacNav similarity to mammalian Na channels. The selected BacNav orthologs will be characterized into three groups based on their voltage dependency: 1) overly hyperpolarized, 2) overly depolarized, and 3) physiological dependency. For the first two groups, the channels will be engineered (via mutagenesis) to shift the voltage dependency curve such that they can be activated within physiologically relevant voltage range. Based on previous studies of BacNa$_V$ structure and biophysics, the voltage dependency properties of these candidates can be altered through site-directed mutagenesis in the highly conserved regions such as activation gate, C-terminal domain (CTD), and intracellular negative charge cluster (INC) or extracellular negative charge cluster (ENC) of voltage-sensing domain (VSD). See e.g., Blanchet et al. (2007), "Acidic residues on the voltage-sensor domain determine the activation of the NaChBac sodium channel," Biophys J, 92(10): p. 3513-23; Shaya et al. (2014), "Structure of a prokaryotic sodium channel pore reveals essential gating elements and an outer ion binding site common to eukaryotic channels," J Mol Biol, 426(2): p. 467-83; Shimomura et al. (2011), "Arrangement and mobility of the voltage sensor domain in prokaryotic voltage-gated sodium channels," J Biol Chem, 286(9): p. 7409-17; Nguyen et al. (2016), "Engineering prokaryotic channels for control of mammalian tissue excitability," Nat Commun, 7: p. 13132. The mutant plasmids will be used to make lentiviruses to transduce Kir2.1+Cx43 expressing HEK293 (293KC) cells. The 293KC cells with >80% transduction efficiency will be seeded on coverslips as confluent monolayers for optical mapping or as single cells for patch clamp studies. Channels that result in action potential propagation while maintaining fast kinetics will be identified as group 4. Channels from group 3 and 4 will be engineered to further speed up BacNa$_V$ activation kinetics and achieve additional improvement in conduction velocity (CV) of action potentials.

Mutations will be generated in the "hinge" of the S6 segment and to change the hydrophobicity of the critical residues on the S2 and S3 transmembrane segments (similar to L64 on Na$_V$SheP), as described in previous studies (Irie et al. (2010), "Comparative study of the gating motif and C-type inactivation in prokaryotic voltage-gated sodium channels," J Biol Chem, 285(6): p. 3685-94; Lacroix et al. (2013), "Molecular bases for the asynchronous activation of sodium and potassium channels required for nerve impulse generation," Neuron, 79(4): p. 651-7). The final BacNav mutants that give rise to highest CV in 293KC monolayer will be characterized and validated in 2-dimensional cultures of neonatal rat ventricular myocytes (NRVMs). MHCK7:BacNa$_V$-GFP lentivirus will be applied to NRVMs on Day 3 (post-isolation). On Day 7: 1) the functional changes induced by lentiviral BacNa$_V$ expression will be assessed via whole-cell patch clamp and optical mapping and 2) structural and molecular changes in NRVMs will be examined via immunostaining and quantitative polymerase chain reaction (qPCR). Use of a lentivirus expressing only GFP will serve as control.

The electrophysiological properties of the mutated channels will be analyzed upon transfection in a Kir2.1+Cx43 expressing HEK293 cell line (293KC) via whole-cell patch clamp. Site-directed mutagenesis will be performed to shift their voltage dependency. The functional BacNa$_V$ channels will be lentivirally transduced in 293KC cells. To test channel function, NRVM will transduce with BacNa$_V$-LV and generate 3-dimensional tissue patches on the day of isolation as previously described (Jackman et al. (2018), "Long-term contractile activity and thyroid hormone supplementation produce engineered rat myocardium with adult-like structure and function," Acta Biomater, 78: p. 98-110). The tissue patches will be cultured for two weeks and then isometric contractile force and conduction velocity will be measured as previously described (Jackman et al. (2018), "Long-term contractile activity and thyroid hormone supplementation produce engineered rat myocardium with adult-like structure and function," Acta Biomater, 78: p. 98-110). Additionally, tissue structure and related gene expression will be compared through immunostaining and qPCR at two-week time point.

### 5.3 Example 3. BacNav mutagenesis and characterization - Trafficking

Recent studies have suggested that there are several pools of Na$_V$1.5 channels that reside in specific subcellular membrane compartments of the cardiomyocyte (i.e. lateral membrane, intercalated disk (ID); Shy et al. (2013), "Cardiac sodium channel NaV1.5 distribution in myocytes via interacting proteins: the multiple pool model," Biochim Biophys Acta, 1833(4): p. 886-94; Rougier, et al. (2019), "A Distinct Pool of Nav1.5 Channels at the Lateral Membrane of Murine Ventricular Cardiomyocytes," Front Physiol, 10: p. 834. Although the specific functions of these distinct pools are still unknown, based on detubulation experiments and studies in *mdx* models, one may estimate that Na$_V$1.5 in T-tubules and lateral membrane are responsible for ≈30% and ≈20% of the sodium current (I$_{Na}$), respectively, and channels at IDs accounts for the remaining 50% I$_{Na}$ (Shy et al. (2014), "PDZ domain-binding motif regulates cardiomyocyte compartment-specific NaV1.5 channel expression and function," Circulation, 130(2): p. 147-60; Bhargava et al. (2013), "Super-resolution scanning patch clamp reveals clustering of functional ion channels in adult ventricular myocyte," Circ Res, 112(8): p. 1112-1120; Orchard and Brette (2008), "t-Tubules and sarcoplasmic reticulum function in cardiac ventricular myocytes," Cardiovasc Res, 7(2): p. 237-44).

The localization of cardiac Na$_V$1.5 channel to these specific sarcolemmal regions is achieved via interactions with a variety of proteins (Rook et al. (2012), "Biology of cardiac sodium channel Nav1.5 expression. Cardiovasc Res," 93(1): p. 12-23). These interactions are facilitated via various motifs displayed by Nav 1.5, including motif VPIAVAESD (SEQ ID NO: 68) at its DII-DIII intracellular loop that binds to Ankyrin-G and the PDZ-binding motif (SIV, SEQ ID NO: 66) at its C-terminus and motif SLA (SEQ ID NO: 67) at N-terminus that bind to syntrophin and SAP97. See e.g., Shy et al. (2014), "PDZ domain-binding motif regulates cardiomyocyte compartment-specific NaV1.5 channel expression and function," Circulation, 130(2): p. 147-60; Petitprez et al. (2011), "SAP97 and dystrophin macromolecular complexes determine two pools of cardiac sodium channels Nav1.5 in cardiomyocytes," Circ Res, 108(3): p. 294-304; Makara, et al. (2014), "Ankyrin-G coordinates intercalated disc signaling platform to regulate cardiac excitability in vivo," Circ Res, 115(11): p. 929-38; Matamoros et al. (2016), "Nav1.5 N-terminal domain binding to alpha1-syntrophin increases membrane density of human Kir2.1, Kir2.2 and Nav1.5 channels," Cardiovasc Res, 110(2): p. 279-90. The immunostaining from our preliminary *in vivo* studies (performed for the previously described BacNav; see Example 4, below) suggests the lack of expression of HA-tagged BacNa$_V$ at both lateral membrane and intercalated disk. Therefore, these motifs will be added to appropriate positions (either at the N-terminus or C-terminus end) in BacNa$_V$ sequence to further improve their membrane expression and compartmentalization in cardiomyocytes and enable targeting of these channels to intended regions such as lateral membrane or intercalated disk.

In addition to specific membrane targeting, improved membrane expression of BacNa$_V$ could also be achieved via speeding up transport along the secretory pathway. A recent study suggests Nav1.5 interacts with adaptor protein complex (AP) 1 and pre-assembles into macromolecular complex with inward rectifier potassium channel, K$_{ir}$2.1, early in the forward trafficking pathway (Ponce-Balbuena et al. (2018), "Cardiac Kir2.1 and NaV1.5 Channels Traffic Together to the Sarcolemma to Control Excitability," Circ Res, 122(11): p. 1501-1516). The BacNa$_V$ transportation could be improved by incorporation of the K$_{ir}$2.1 trafficking motifs such as the Golgi export sequence trafficking signal (KSRITSEGEYIPLDQIDINV, SEQ ID NO: 63), the endoplasmic reticulum (ER) export sequence (FCYENEV, SEQ ID NO: 62), and potassium channel PDZ-binding motif (SEI, SEQ ID NO: 65) (Hofherr et al. (2005), "Selective Golgi export of Kir2.1 controls the stoichiometry of functional Kir2.x channel heteromers," J Cell Sci, 118(Pt 9): p. 1935-43; Leonoudakis et al. (2004), "Protein trafficking and anchoring complexes revealed by proteomic analysis of inward rectifier potassium channel (Kir2.x)-associated proteins," J Biol Chem, 2004. 279(21): p. 22331-46; Gong et al. (2014), "Imaging neural spiking in brain tissue using FRET-opsin protein voltage sensors," Nat Commun, 5: p. 3674). Moreover, adding specific tyrosine-based sorting signal (YSVL, SEQ ID NO: 69) could mark BacNa$_v$ channels for incorporation into clathrin-coated vesicles at the *trans*-Golgi similar to Kir2.1 and conferring specificity on membrane trafficking (Park and Guo, "Adaptor protein complexes and intracellular transport," Biosci Rep, 34(4)).

Membrane targeting can also be achieved by introducing additional posttranslational modification (PTM) for lipid modifications, which often direct proteins to the cell membrane. Several types of lipid modifications have been identified while a few motifs have been used repeatedly for membrane targeting including: prenylation (farnesylation and geranylgeranylation), N-myristoylation, S-palmitoylation, and glycosylphosphatidylinositol (GPI) anchoring (Haucke et al. (2007), "Lipids and lipid modifications in the regulation of membrane traffic," Curr Opin Cell Biol, 19(4): p. 426-35). One of the most widely used prenylation motifs is motif $CX_1X_2X_3$ (SEQ ID NO: 70; $X_1$ = aliphatic, $X_2$ = aliphatic, $X_3$ = any amino acid) at the C-terminus, which will introduce a triplet of PTMs to make protein hydrophobic at their C-termini to facilitate its association with membranes (Hancock et al. (1991), "A CAAX or a CAAL motif and a second signal are sufficient for plasma membrane targeting of ras proteins," EMBO J, 10(13): p. 4033-9). Addition of a myristoylation sequence (MGXXXSXX, SEQ ID NO: 74; X = any amino acid) to the N-terminus Gly residue will result in the attachment of myrostic acid to N- terminal Gly residue though an amide linkage (Hayashi and Titani, (2010), "N-myristoylated proteins, key components in intracellular signal transduction systems enabling rapid and flexible cell responses," Proc Jpn Acad Ser B Phys Biol Sci, 86(5): p. 494-508). The N-myristoylation modification has no consensus sequence, but it requires a glycine at position 2 (after the initiator methionine), amino terminal basic residues, and a Ser at position 6 (Hayashi and Titani, (2010), "N-myristoylated proteins, key components in intracellular signal transduction systems enabling rapid and flexible cell responses," Proc Jpn Acad Ser B Phys Biol Sci, 86(5): p. 494-508).

Incorporation of the dual palmitoylation signal from the GAP-43 gene (MLCCMRRTKQ, SEQ ID NO: 71) has been shown to target the tagged protein to the membrane with palmitoylation at the Cys residues at positions 3 and 4 (Zuber et al. (1989), "A membrane-targeting signal in the amino terminus of the neuronal protein GAP-43," Nature, 341(6240): p. 345-8). In contrast, the addition of a GPI anchor occurs in the endoplasmic reticulum, where a GPI transamidase links the GPI unit and the C-terminus of a protein through amide linkage. The GPI anchor facilities protein attachment to the outer leaflet of the cell membrane and the structure has been identified in many eukaryotic proteins. The GPI anchor is a complex structure and some most commonly used ones include the C-terminal sequence derived from the Thy-1 N-terminal GPI-linked signal sequence (KDNTTLQEFATLAN; SEQ ID NO: 72), and GPI-attachment signal of lymphocyte-function-associated antigen 3 (PSSGHSRYALI; SEQ ID NO: 73) (Rhee et al. (2006), "In vivo imaging and differential localization of lipid-modified GFP-variant fusions in embryonic stem cells and mice," Genesis, 44(4): p. 202-18; Harrison et al. (1994), "A convenient method for the construction and expression of GPI-anchored proteins," Nucleic Acids Res, 22(18): p. 3813-4).

To maximize the channel expression, the above mentioned trafficking motifs will be added to the BacNa$_v$ C- or N-terminus to target engineered BacNa$_v$ to specific subcellular membrane compartments of the cardiomyocyte and improve the overall expression of BacNa$_v$. Lentivirus encoding different constructs (pRRL-MHCK7-HA-BacNav-motif or pRRL-MHCK7-HA-motif-BacNav) will be produced and tested in NRVM monolayers and cultured adult mouse heart slices. The constructs that result in higher BacNav membrane expression and sodium current density will be selected and packaged into AAV for tail-vein injection in mice. The adult cardiomyocytes will be isolated through Langendorff retrograde perfusion 4 weeks following injection and the channel trafficking and expression will be analyzed using immunostaining and whole-cell patch clamp.

### 5.4 Example 4. *In vivo* evaluation of prokaryotic channel-based gene therapy for cardiac infarction and arrhythmias

The novel BacNav and BacCav channels will be packaged into AAV vectors to perform gene therapy studies in rodent models of heart disease. It will be assessed if intravenous AAV9 MHCK7-BacNav administration in mice can improve sodium current density, impaired conduction, and arrhythmogenesis in heterozygous Scn5a-knockout mice (Brugada syndrome mice) using the inventors' established electrophysiology tests. See e.g., Nguyen et al. (2016), "Engineering prokaryotic channels for control of mammalian tissue excitability," Nat Commun., 7:13132; Jackman et al. (2018), "Engineered cardiac tissue patch maintains structural and electrical properties after epicardial implantation," Biomaterials, 159:48-58; Shadrin et al. (2017), "Cardiopatch platform enables maturation and scale-up of human pluripotent stem cell-derived engineered heart tissues. Nat Commun., 8:1825; Wang et al. (2011), "Fibroblast growth factor homologous factor 13 regulates Na+ channels and conduction velocity in murine hearts," Circ Res., 109:775-82; and Zhang et al. (2015), "STIM1-Ca2+ signaling modulates automaticity of the mouse sinoatrial node," Proc Natl Acad Sci USA, 112(41), E5618-27. Adult cardiomyocytes isolated from BacNav-administered animals will be assessed for the channel expression and electrophysiological changes using immunostaining and whole-cell patch clamp, respectively. BacNav therapy will also be evaluated in rat myocardial infarction model *in vivo* and during global cardiac ischemia induced by halting heart perfusion on the Landgendorff apparatus ex vivo. It will be assessed if BacNav therapy can improve impaired action potential conduction and programmed pacing-induced arrhythmogenesis using optical mapping as well as if it can improve cardiac contractile function using echocardiography. *In vivo* studies on the newly identified BacNavs will be conducted similarly as those performed on previously identified BacNav SheP (described in U.S. Patent Publication No. US 2019/0030186A1). A brief description of the SheP BacNav experiments is provided below.

<u>Approach.</u> Intravenous injection of self-complementary AAV serotype 9 (scAAV9) vector was chosen as gene delivery method as it allows global and uniform gene expression throughout the heart one week after tail-vein injection. Male 6-10 week old CD-1 mice (Charles River Laboratories) were injected via the tail vein with 200 $\mu$l of AAV9 solution (1 x $10^{11}$ vg/mouse for scAAV9-CAG-GFP, 5 x $10^{11}$ vg/mouse for scAAV9-MHCK7-h2SheP-HA, and 2 x $10^{12}$ vg/mouse for AAV9-CAG-h2SheP-GFP). Mice were euthanized by isofluorane inhalation at 1-2 weeks (for self-complementary AAVs) or 4 weeks (for single-stranded AAVs) post injection and the hearts were harvested for cardiomyocyte isolation or histology. Adult mouse ventricular cardiomyocytes were isolated and cultured according to a previously published Langendorff-free procedure (Li et al. (2014), "Isolation and culture of adult mouse cardiomyocytes for cell signaling and in vitro cardiac hypertrophy," J Vis Exp, (87). Isolated cells were plated onto 21 mm-diameter Aclar (Ted Pella) coverslips coated with laminin (5 $\mu$g/mL, Thermo Fisher Scientific) and incubated for 4 h at 37 °C before patch clamp studies.

<u>AAV production and titration.</u> All recombinant AAV viruses were generated using the standard triple transfection method. Briefly, 293T cells (Takara, 632180) were co-transfected with the adenoviral helper plasmid pALD-X80 (Aldevron), the packaging plasmid AAV2/9 (gift from James M. Wilson, Addgene plasmid #112865), and the transfer ITR plasmid (1:1:1 molar ratios) using polyethylenimine (PEI) 40K Max transfection reagent (Polysciences). Transfected cells were supplied with fresh media 48 - 72 hr after transfection and both cells and supernatant containing virus particles were collected 120 hr after transfection. Collected cells were centrifuged (500 g, 10 min) and cell pellet was resuspended in cell lysis buffer (0.15 M NaCl + 0.05 M Tris-HCl, pH 8.5) and lysed through four sequential freeze-thaw cycles (15 min in dry ice/ethanol bath followed by 5 min in 37 °C water bath). AAV-containing cell lysate was collected following centrifugation at 4,500 rpm and 4 °C for 30 min to remove cell debris. Collected media supernatant was filtered through 0.45 mm cellulose acetate filter (Corning) before being combined with 40% polyethylene glycol (PEG) solution at 4:1 volume ratio for overnight incubation at 4 °C. Concentrated AAV particles were harvested following 15 min centrifugation (2,818 g, 4 °C), resuspended in cell lysis buffer, and combined with viral particles collected from cell pellet. Benzonase (Millipore Sigma) was added to the virus-containing solution at a final concentration of 50 U/ml with subsequent incubation at 37 °C for 30 min. Viral particles were purified via iodixanol density gradient ultracentrifugation at 30,000 rpm and 17 °C for 15-17 hours (WX Ultra 80, Thermo Fisher Scientific). Fractions containing AAV9 were collected and subjected to subsequent phosphate-buffered saline (PBS) buffer exchange using Zeba Spin (40-kDa-molecular-weight cutoff [MWCO]) desalting columns (Thermo Fisher Scientific). Viral titers of purified viruses were determined by quantitative PCR with primers that specifically amplify the AAV2 ITR regions.

<u>Immunostaining.</u> Cell monolayers were fixed in 4% paraformaldehyde (PFA) for 10 min at room temperature. Hearts were fixed in 4% PFA at 4 °C overnight, immersed in 30% (w/v) sucrose for cryoprotection, embedded and frozen in OCT compound (VWR) using liquid nitrogen, and cut into 10 $\mu$m sections using cryostat (Leica). Fixed monolayers or heart sections were permeabilized and blocked in blocking solution (5% chicken serum + 0.1% Triton-X, 30 min). The following primary antibodies (1 hour incubation) were used: anti-sarcomeric $\alpha$-actinin (Sigma, a7811, 1:200), anti-vimentin (Abcam, ab92547, 1:500), anti-cardiac troponin T (Sigma, ab45932, 1:200), and anti-HA tag (Cell Signaling Technology, C29F4, 1:200). Secondary antibodies (1 hour incubation) included: Alexa Fluor 488 (Thermo Fisher Scientific, A-21200 or A-21441, 1:200), Alexa Fluor 594 (Thermo Fisher Scientific, A-21201 or A-21442, 1:200), Alexa Fluor 647 (Thermo Fisher Scientific, A-21463, 1:200), Alexa Fluor 488-conjugated phalloidin (Thermo Fisher Scientific, A12379, 1:300), Alexa Fluor 647-conjugated phalloidin (Thermo Fisher Scientific, A22287, 1:300), and DAPI (Sigma, 1:300). All immunostaining steps were performed at room temperature. Fluorescence images were acquired using inverted fluorescence (Nikon TE2000) or confocal (Leica SP5) microscope, and processed with ImageJ software.

An important step toward the development of future therapeutic applications involving BacNav is the validation of their functionality *in vivo*. To achieve this goal, trafficking and function of the SheP BacBav channel was examined in adult cardiomyocytes following *in vivo* delivery. Intravenous injection of self-complementary AAV serotype 9 (scAAV9) vector was chosen as gene delivery method as it allows global and uniform gene expression throughout the heart one week after tail-vein injection. To investigate sarcolemmal expression and distribution of the SheP BacNav in transduced hearts, scAAV9-MHCK7-h2SheP-HA virus was administered and 7 days later, via immunostaining for the HA tag, it was observed that the channels were targeted to the T-tubular membrane, known to be rich in endogenous ion channels and transporters. Furthermore, patch clamp recordings in dissociated cardiomyocytes showed the presence of the SheP BacBav current with characteristic peak I-V relationship in cells transduced with h2SheP AAV9 but not in non-transduced cells from the same hearts.

Similar results are expected in the testing of the novel BacNav sodium channel polypeptides provided in this disclosure.

All patents, patent publications, patent applications, journal articles, books, technical references, and the like discussed in the instant disclosure are incorporated herein by reference in their entirety for all purposes.

It is to be understood that the figures and descriptions of the disclosure have been simplified to illustrate elements that are relevant for a clear understanding of the disclosure. It should be appreciated that the figures are presented for illustrative purposes and not as construction drawings. Omitted details and modifications or alternative embodiments are within the purview of persons of ordinary skill in the art.

It can be appreciated that, in certain aspects of the disclosure, a single component may be replaced by multiple components, and multiple components may be replaced by a single component, to provide an element or structure or to perform a given function or functions. Except where such substitution would not be operative to practice certain embodiments, such substitution is considered within the scope of the disclosure.

The examples presented herein are intended to illustrate potential and specific implementations of the invention. It can be appreciated that the examples are intended primarily for purposes of illustration for those skilled in the art. There may be variations to these diagrams or the operations described herein without departing from the spirit of the invention. For instance, in certain cases, method steps or operations may be performed or executed in differing order, or operations may be added, deleted or modified.

Different arrangements of the components depicted in the drawings or described above, as well as components and steps not shown or described are possible. Similarly, some features and sub-combinations are useful and may be employed without reference to other features and sub-combinations. Aspects and embodiments of the invention have been described for illustrative and not restrictive purposes, and alternative embodiments will become apparent to readers of this patent. Accordingly, the present invention is not limited to the embodiments described above or depicted in the drawings, and various embodiments and modifications can be made without departing from the scope of the claims below.

While exemplary embodiments have been described in some detail, by way of example and for clarity of understanding, those of skill in the art will recognize that a variety of modification, adaptations, and changes may be employed. Hence, the scope of the present invention should be limited solely by the claims.

## Claims

1. A nucleic acid comprising a nucleotide sequence encoding a sodium channel polypeptide comprising an amino acid sequence having at least 90% identity to the sequence of SEQ ID NO: 12 or SEQ ID NO: 6, wherein the nucleotide sequence is operatively linked to a heterologous promoter.

2. A nucleic acid comprising a nucleotide sequence encoding a sodium channel polypeptide comprising an amino acid sequence having at least 90% identity to the sequence of SEQ ID NO: 12 or SEQ ID NO: 6, wherein the nucleotide sequence is not a naturally occurring sequence encoding the sodium channel polypeptide.

3. The nucleic acid of claim 1 or 2, wherein:

   (a) the nucleotide sequence is codon optimized for expression of the sodium channel polypeptide in human cells;
   (b) the nucleotide sequence is SEQ ID NO: 41 or SEQ ID NO: 35;
   (c) the nucleotide sequence comprises at least one of a sequence encoding a motif, wherein the sequence encoding a motif is selected from the group consisting of SEQ ID NOs: 59-74; and/or
   (d) the nucleic acid comprises a sequence encoding at least one of a potassium channel and/or a connexin protein.

4. A vector comprising the nucleic acid of any one of claims 1 to 3, optionally wherein the vector is a viral vector, optionally wherein the viral vector is a an adeno-associated viral (AAV) vector, a lentiviral vector, or a retroviral vector.

5. A virus comprising the nucleic acid of any one of claims 1 to 3 or the vector of claim 4, optionally wherein the virus is an AAV, a lentivirus, or a retrovirus.

6. A cell comprising the nucleic acid of claim 1 to 3, the vector of claim 4, or the virus of claim 5, optionally wherein the cell is a mammalian cell.

7. A pharmaceutical composition comprising the nucleic acid of claim 1 to 3, the vector of claim 4, the virus of claim 5, or the cell of claim 6, and a pharmaceutically acceptable excipient.

8. A tissue patch comprising a plurality of cells of claim 6.

9. An agent for use in a method of treating a cardiac condition **characterized by** impaired action potential conduction in the heart, said method comprising administering to a subject in need thereof a therapeutically effective amount of said agent, wherein said agent is the nucleic acid of claim 1 to 3, the vector of claim 4, the virus of claim 5, the cell of claim 6, or the pharmaceutical composition of claim 7.

10. An agent for use in a method of treating a cardiac condition **characterized by** impaired action potential conduction in the heart, said method comprising implanting in a subject in need thereof said agent onto the surface of a cardiac muscle of the subject, wherein said agent is implanted on an area of the cardiac muscle having impaired action potential conduction, wherein said agent is the tissue patch of claim 8.

11. The agent for use according to claim 9 or claim 10, wherein the cardiac condition is one or more of cardiac arrhythmia, atrial fibrillation, ventricular fibrillation, atrioventricular block, ventricular tachycardia, heart failure, damage from myocardial infarction, damage from stroke, brugada syndrome, left bundle branch block, or chronic ischemia.

12. An agent for use in a method of treating a central nervous system (CNS) disorder, a peripheral nervous system (PNS) disorder, or a skeletal muscle disorder, said method comprising administering to a subject in need thereof a therapeutically effective amount of the nucleic acid of claim 1 to 3, the vector of claim 4, the virus of claim 5, the cell of claim 6, or the pharmaceutical composition of claim 7.

13. The agent for use according to any one of claims 9 to claim 12, wherein the subject is a human.

14. An *in vitro* method of increasing the conduction of a cell comprising introducing the nucleic acid of claim 1 to 3, the vector of claim 4, or the virus of claim 5, wherein introduction of the nucleic acid, the vector, or the virus induces the expression of the sodium channel polypeptide, thereby increasing conduction of the cell, optionally wherein the cell is a mammalian cell and/or wherein the cell is a cardiomyocyte.

15. A method of producing a tissue patch comprising

(i) seeding a plurality of cells of claim 6 on a solid support, thereby forming a cell-seeded construct; and
(ii) culturing the cell-seeded construct in a culture medium for a period of time, thereby producing the tissue patch.

Fig. 1A

EP 4 450 514 A2

**Fig. 1B**

EP 4 450 514 A2

V1/2 of activation (mV)

50　25　0　-25　-50　-75　-100

h2Shep, BacNav 3, BacNav 4, BacNav 5, BacNav 6, BacNav 7, BacNav 10, BacNav 11, BacNav 12, BacNav 13, BacNav 16, BacNav 18, BacNav 19, BacNav 20, BacNav 24, BacNav 25, BacNav 26, BacNav 28, BacNav 29, BacNav 31, BacNav 32, BacNav35, BacNav 36, BacNav 37, BacNav 38, BacNav 40, BacNav 42, BacNav 44, BacNav 46, BacNav 50

Fig. 1C

Fig. 2A

Fig. 2B

Fig. 2C

**Fig. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62914722
- WO 2005033321 A
- US 6596535 B
- US 7125717 B
- US 7456683 B
- US 7790449 B
- US 7588772 B
- US 6001650 A
- US 7629322 B
- US 6764845 B
- US 4963489 A
- US 10106776 B
- US 20190030186 A1

**Non-patent literature cited in the description**

- **PAYANDEH, J. ; MINOR, D.L.** *J. Mol. Biol.,* 2015, vol. 427 (1), 3-30
- **ZHANG et al.** Protein engineering with unnatural amino acids. *Curr. Opin. Struct. Biol.,* 2013, vol. 23 (4), 581-587
- **XIE.** *Adding amino acids to the genetic repertoire,* 2005, vol. 9 (6), 548-54
- **SMITH ; WATERMAN.** *Add. APL. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. (U.S.A.),* 1988, vol. 85, 2444
- **ALTSCHUL et al.** *J. Mol. Biol.,* 1990, vol. 215, 403-410
- **ALTSCHUL et al.** *Nucleic Acids Res.,* 1977, vol. 25, 3389-3402
- **KARLIN ; ALTSCHUL.** *Proc. Nat'l. Acad. Sci. USA,* 1993, vol. 90, 5873-5787
- **WELCH et al.** Design parameters to control synthetic gene expression in Escherichia coli. *PloS one,* 2009, vol. 4, e7002
- **RAAB et al.** The GeneOptimizer Algorithm: using a sliding window approach to cope with the vast sequence space in multiparameter DNA sequence optimization. *Syst Synth Biol,* 2010, vol. 4, 215
- **RIVERA.** Long-term pharmacologically regulated expression of erythropoietin in primates following AAV-mediated gene transfer. *Blood,* 2005, vol. 105 (4), 1424-1430
- **SALVA et al.** Design of tissue-specific regulatory cassettes for high-level rAAV-mediated expression in skeletal and cardiac muscle. *Mol Ther,* 2007, vol. 15, 320-329
- **AMOASII et al.** Single-cut genome editing restores dystrophin expression in a new mouse model of muscular dystrophy. *Sci Transl Med,* 2017, vol. 9, 418
- **PRASAD et al.** Robust cardiomyocyte-specific gene expression following systemic injection of AAV: in vivo gene delivery follows a Poisson distribution. *Gene Ther,* 2011, 43-52
- **WERFEL et al.** Rapid and highly efficient inducible cardiac gene knockout in adult mice using AAV-mediated expression of Cre recombinase. *Cardiovasc Res,* 2014, vol. 104 (1), 15-23
- **PHILLIPS et al.** Vigilant vector: heart-specific promoter in an adeno-associated virus vector for cardioprotection. *Hypertension,* 2002, vol. 39 (2), 651-5
- **GULICK et al.** Isolation and characterization of the mouse cardiac myosin heavy chain genes. *J Biol Chem,* 1991, vol. 266 (14), 9180-5
- **NI et al.** Atrial-Specific Gene Delivery Using an Adeno-Associated Viral Vector. *Circ Res,* 2019, vol. 124 (2), 256-262
- **HOFHERR et al.** Selective Golgi export of Kir2.1 controls the stoichiometry of functional Kir2.x channel heteromers. *J Cell Sci,* 2005, vol. 118 (9), 1935-43
- **LEONOUDAKIS.** Protein trafficking and anchoring complexes revealed by proteomic analysis of inward rectifier potassium channel (Kir2.x)-associated proteins. *J Biol Chem,* 2004, vol. 279 (21), 22331-46
- **GONG et al.** Imaging neural spiking in brain tissue using FRET-opsin protein voltage sensors. *Nat Commun,* 2014, vol. 5
- **PARK ; GUO.** Adaptor protein complexes and intracellular transport. *Biosci Rep,* vol. 34 (4
- **HAUCKE et al.** Lipids and lipid modifications in the regulation of membrane traffic. *Curr Opin Cell Biol,* 2007, vol. 19 (4), 426-35

- **HANCOCK et al.** A CAAX or a CAAL motif and a second signal are sufficient for plasma membrane targeting of ras proteins. *EMBO J,* 1991, vol. 10 (13), 4033-9
- **HAYASHI ; TITANI.** N-myristoylated proteins, key components in intracellular signal transduction systems enabling rapid and flexible cell responses. *Proc Jpn Acad Ser B Phys Biol Sci,* 2010, vol. 86 (5), 494-508
- **LI ; GALLIN.** VKCDB: "Voltage-gated potassium channel database. *BMC Bioinformatics,* 2004, vol. 5, 3
- **SCHAK NIELSEN, M. et al.** Gap Junctions. *Compr. Physiol.,* 2012, vol. 2 (3), 1981-2035
- **SAMBROOK et al.** Molecular Cloning: A Laboratory Manual. Cold Spring Harbor Laboratory Press, 1992
- Advanced Bacterial Genetics. Cold Spring Harbor Laboratory Press, 1980
- **WANG et al.** Adeno-associated virus vector as a platform for gene therapy delivery. *Nat Rev Drug Discov,* 2019, vol. 18, 358-378
- **LI et al.** Engineering adeno-associated virus vectors for gene therapy. *Nat Rev Genet,* 2020, vol. 21, 255-272
- **DUNBAR et al.** Gene therapy comes of age. *Science,* 2018, vol. 359, 6372
- **PENAUD-BUDLOO et al.** Pharmacology of recombinant Adeno-Associated Virus production. *Mol Ther Meth Clin Dev,* 2018, vol. 8, 166-180
- **GONÇALVES, M.A.** Adeno-associated virus: from defective virus to effective vector. *Virol J,* 2005, vol. 2, 43
- **MCCARTY et al.** Self-complementary AAV vectors: advances and applications. *Mol Ther,* 2008, vol. 16, 1648-1656
- **BERNS K.** Parvovirus replication. *Microbiol Rev,* 1990, vol. 54, 316-329
- **LING et al.** Enhanced transgene expression from recombinant single-stranded D-sequence-substituted adeno-associated virus vectors in human cell lines in vitro and in murine hepatocytes in vivo. *J Virol,* 2015, vol. 89 (2), 952-961
- **TISCORNIA et al.** Production and purification of lentiviral vectors. *Nature Protocols,* 2006, vol. 1, 241-245
- **KEEKER et al.** Gene Therapy 2017: Progress and Future Directions. *Clin Transl Sci.,* 2017, vol. 10, 242-248
- **HIGUCHI et al.** Direct Injection of Kit Ligand-2 Lentivirus Improves Cardiac Repair and Rescues Mice Post-Myocardial Infarction. *Molecular Therapy,* 2009, vol. 17 (2), 262-68
- **ZHAO et al.** Lentiviral Vectors for Delivery of Genes into Neonatal and Adult Ventricular Cardiac Myocytes in Vitro and in Vivo. *Basic Research in Cardiology,* 2002, vol. 97 (5), 348-58
- **ZOLOTUKIN et al.** Production And Purification Of Serotype 1, 2, And 5 Recombinant Adeno-Associated Viral Vectors. *Methods,* 2002, vol. 28, 158-167
- **LI et al.** Engineering adeno-associated virus vectors for gene therapy. *Nat Rev Genet,* 2020, vol. 21, 255-272
- **SAMBROOK et al.** Molecular Cloning: A Laboratory Manual. Cold Spring Harbor Press
- **GRAHAM et al.** *Virol,* 1973, vol. 52, 456
- **DAVIS et al.** Basic Methods in Molecular Biology. Elsevier, 1986
- **CHU et al.** *Gene,* 1981, vol. 13, 197
- **WANG ; MCMANUS.** Lentivirus production. *J. Vis. Exp.,* 2009, vol. 32, 1499
- **VIGNA ; NALDINI.** Lentiviral vectors: excellent tools for experimental gene transfer and promising candidates for gene therapy. *J Gene Med.,* 2000, vol. 2, 308-316
- **SEGURA et al.** New protocol for lentiviral vector mass production. *Methods Mol Biol.,* 2010, vol. 614, 39-52
- **NGUYEN et al.** Generation and customization of biosynthetic excitable tissues for electrophysiological studies and cell-based therapies. *Nat Protoc,* 2018, vol. 13 (5), 927-945
- **ITAKA ; KATAOKA.** Recent development of nonviral gene delivery systems with virus-like structures and mechanisms. *Eur J Pharma and Biopharma,* 2009, vol. 71, 475-483
- **KEELER et al.** Gene Therapy 2017: Progress and Future Directions. *Clin. Transl. Sci.,* 2017, vol. 10, 242-248
- **FRESHNEY.** Culture of Animal Cells, a Manual of Basic Technique. Wiley-Liss, 1994
- **DOYLE ; GRIFFITHS.** Mammalian Cell Culture: Essential Techniques. John Wiley and Sons, 1997
- **HUMASON.** Animal Tissue Techniques. W.H. Freeman and Company, 1979
- **RICCIARDELLI et al.** *In vitro Cell Dev. Biol.,* 1989, vol. 25, 1016-1024
- **LI et al.** *Signal Transduction and Targeted Therapy,* 2020, vol. 5
- Remington: The Science and Practice of Pharmacy. Pharmaceutical Press, 2012
- **BENNICELLI et al.** Reversal of blindness in animal models of leber congenital amaurosis using optimized AAV2-mediated gene transfer. *Mol Ther.,* 2008, vol. 16 (3), 458-65
- **LI et al.** Tissue-engineered 3-dimensional (3D) microenvironment enhances the direct reprogramming of fibroblasts into cardiomyocytes by microRNAs. *Scientific Reports,* 2016, vol. 6, 38815
- **JAYAWARDENA et al.** MicroRNA-mediated in vitro and in vivo direct reprogramming of cardiac fibroblasts to cardiomyocytes. *Circ Res,* 2012, vol. 110, 1465-1473

- **JAYAWARDENA et al.** MicroRNA induced cardiac reprogramming in vivo: evidence for mature cardiac myocytes and improved cardiac function. *Circ Res,* 2015, vol. 116, 418-424
- **JAYAWARDENA et al.** Direct reprogramming of cardiac fibroblasts to cardiomyocytes using microRNAs. *Methods Mol Biol,* 2014, vol. 1150, 263-272
- **IEDA et al.** Direct reprogramming of fibroblasts into functional cardiomyocytes by defined factors. *Cell,* 2010, vol. 142, 375-386
- **QIAN et al.** In vivo reprogramming of murine cardiac fibroblasts into induced cardiomyocytes. *Nature,* 2012, vol. 485, 593-598
- **SONG et al.** Heart repair by reprogramming non-myocytes with cardiac transcription factors. *Nature,* 2012, vol. 485, 599-604
- **DOBKIN et al.** Accurate Quantification and Characterization of Adeno-Associated Viral Vectors. *Front Microbiol,* 2019, vol. 10, 1570-1583
- **LOCK et al.** Absolute determination of single-stranded and self-complementary adeno-associated viral vector genome titers by droplet digital PCR. *Hum Gene TherMethods,* 2014, vol. 25, 115-125
- **SOMMER et al.** Quantification of adeno-associated virus particles and empty capsids by optical density measurement. *Mol Ther,* 2003, vol. 7, 122-128
- **GRIMM et al.** Titration of AAV-2 particles via a novel capsid ELISA: packaging of genomes can limit production of recombinant AAV-2. *Gene Ther,* 1999, vol. 6, 1322-1330
- **MAYNARD et al.** Fast-Seq: A Simple Method for Rapid and Inexpensive Validation of Packaged Single-Stranded Adeno-Associated Viral Genomes in Academic Settings. *Hum Gene Ther,* 2019, vol. 30 (6), 195-205
- **PIEDRA et al.** Development of a rapid, robust, and universal picogreen-based method to titer adeno-associated vectors. *Hum Gene Ther Methods,* 2015, vol. 26, 35-42
- **PAPADATOS. G. A. et al.** *PNAS,* 2002, vol. 9, 6210-6215
- **SHY. D. et al.** *Circulation,* 2014, vol. 130, 147-60
- **HAKIM, P. et al.** *Prog. Biophys. Mol. Biol.,* 2008, vol. 98, 251-266
- **MAKARA. M. A. et al.** *Circ. Res.,* 2014, vol. 115, 929-938
- **CERRONE, M. et al.** *Cardiovasc. Res.,* 2012, vol. 95, 460-468
- **GROSSMANN. K. S. et al.** *J. Cell Biol.,* 2004, vol. 167, 149-160
- **RIZZO, S. et al.** *Cardiovasc. Res.,* 2012, vol. 95, 409-418
- **PILICHOU, K. et al.** *J. Exp. Med.,* 2009, vol. 206
- **MEHDI. H. et al.** *Circulation,* 2014, vol. 130, A17871-A17871
- **GODDARD. C. A. et al.** *Acta Physiol. (Oxf).,* 2008, vol. 194, 123-140
- **KING, J. H.. et al.** *Cardiovasc. Res.,* 2013, vol. 99, 751-759
- **HAN. Z. et al.** *PLoS One,* 2015, vol. 10, e0122436
- Principles of Tissue Engineering. Academic Press, 2000
- Methods of Tissue Engineering. Academic Press, 2001
- Animal Cell Culture. Oxford University Press, 2000
- **S. N. MOHAMED et al.** *In Vitro Cell and Develop. Biol.,* 1983, vol. 19, 471-478
- **P. LIBBY.** *J. Mol. Cell. Cardiol.,* 1984, vol. 16, 803-811
- **D. L. FREERKSEN et al.** *J. Cell. Physiol.,* 1984, vol. 120, 126-134
- **G. KESSLER-ICEKSON et al.** *Exp. Cell Res.,* 1984, vol. 155, 113-120
- **J. S. KARLINER et al.** *Biochem. Biophys. Res. Comm.,* 1985, vol. 128, 376-382
- **T. SUZUKI et al.** *FEBS Letters,* 1990, vol. 268, 149-151
- **T. SUZUKI et al.** *J. Cardiov. Pharmacol.,* 1991, vol. 17, S182-S186
- **T. SUZUKI et al.** *J. Mol. Cell. Cardiol.,* 1997, vol. 29, 2087-2093
- **ZHANG et al.** Tissue-engineered cardiac patch for advanced functional maturation of human ESC-derived cardiomyocytes. *Biomaterials,* 2013, vol. 34 (23), 5813-20
- **BIAN et al.** Robust T-tubulation and maturation of cardiomyocytes using tissue-engineered epicardial mimetics. *Biomaterials,* 2014, vol. 35 (12), 3819-28
- **BIAN et al.** Controlling the structural and functional anisotropy of engineered cardiac tissues. *Biofabrication,* 2014, vol. 6 (2), 024109
- **HINDS et al.** The role of extracellular matrix composition in structure and function of bioengineered skeletal muscle. *Biomaterials,* 2011, vol. 32, 3575-3583
- **LIAU et al.** Pluripotent stem cell-derived cardiac tissue patch with advanced structure and function. *Biomaterials,* 2011, vol. 32, 9180-9187
- **BIAN et al.** Local tissue geometry determines contractile force generation of engineered muscle networks. *Tissue Eng. Part A,* 2012, vol. 18, 957-967
- **KLINGER ; BURSAC.** Cardiac cell therapy in vitro: reproducible assays for comparing the efficacy of different donor cells. *IEEE Eng Med Biol Mag,* 2008, vol. 27 (1), 72-80
- **KIRKTON ; BURSAC.** Engineering biosynthetic excitable tissues from unexcitable cells for electrophysiological and cell therapy studie. *Nat Commun,* 2011, vol. 2, 300

- **BURSAC et al.** Multiarm spirals in a two-dimensional cardiac substrate. *Proc Natl Acad Sci USA,* 2004, vol. 101 (43), 15530-4
- **BADIE ; BURSAC.** Novel micropatterned cardiac cell cultures with realistic ventricular microstructure. *Biophys J,* vol. 96 (9), 3873-85
- **BLANCHET et al.** Acidic residues on the voltage-sensor domain determine the activation of the NaChBac sodium channel. *Biophys J,* 2007, vol. 92 (10), 3513-23
- **SHAYA et al.** Structure of a prokaryotic sodium channel pore reveals essential gating elements and an outer ion binding site common to eukaryotic channels. *J Mol Biol,* 2014, vol. 426 (2), 467-83
- **SHIMOMURA et al.** Arrangement and mobility of the voltage sensor domain in prokaryotic voltage-gated sodium channels. *J Biol Chem,* 2011, vol. 286 (9), 7409-17
- **NGUYEN et al.** Engineering prokaryotic channels for control of mammalian tissue excitability. *Nat Commun,* 2016, vol. 7, 13132
- **IRIE et al.** Comparative study of the gating motif and C-type inactivation in prokaryotic voltage-gated sodium channels. *J Biol Chem,* 2010, vol. 285 (6), 3685-94
- **LACROIX et al.** Molecular bases for the asynchronous activation of sodium and potassium channels required for nerve impulse generation. *Neuron,* 2013, vol. 79 (4), 651-7
- **JACKMAN et al.** Long-term contractile activity and thyroid hormone supplementation produce engineered rat myocardium with adult-like structure and function. *Acta Biomater,* 2018, vol. 78, 98-110
- **SHY et al.** Cardiac sodium channel NaV1.5 distribution in myocytes via interacting proteins: the multiple pool model. *Biochim Biophys Acta,* 2013, vol. 1833 (4), 886-94
- **ROUGIER et al.** A Distinct Pool of Nav1.5 Channels at the Lateral Membrane of Murine Ventricular Cardiomyocytes. *Front Physiol,* 2019, vol. 10, 834
- **SHY et al.** PDZ domain-binding motif regulates cardiomyocyte compartment-specific NaV1.5 channel expression and function. *Circulation,* 2014, vol. 130 (2), 147-60
- **BHARGAVA et al.** Super-resolution scanning patch clamp reveals clustering of functional ion channels in adult ventricular myocyte. *Circ Res,* 2013, vol. 112 (8), 1112-1120
- **ORCHARD ; BRETTE.** t-Tubules and sarcoplasmic reticulum function in cardiac ventricular myocytes. *Cardiovasc Res,* 2008, vol. 7 (2), 237-44
- **ROOK et al.** Biology of cardiac sodium channel Nav1.5 expression. *Cardiovasc Res,* 2012, vol. 93 (1), 12-23
- **PETITPREZ et al.** SAP97 and dystrophin macromolecular complexes determine two pools of cardiac sodium channels Nav1.5 in cardiomyocytes. *Circ Res,* 2011, vol. 108 (3), 294-304
- **MAKARA et al.** Ankyrin-G coordinates intercalated disc signaling platform to regulate cardiac excitability in vivo. *Circ Res,* 2014, vol. 115 (11), 929-38
- **MATAMOROS et al.** Nav1.5 N-terminal domain binding to alpha1-syntrophin increases membrane density of human Kir2.1, Kir2.2 and Nav1.5 channels. *Cardiovasc Res,* 2016, vol. 110 (2), 279-90
- **PONCE-BALBUENA et al.** Cardiac Kir2.1 and NaV1.5 Channels Traffic Together to the Sarcolemma to Control Excitability. *Circ Res,* 2018, vol. 122 (11), 1501-1516
- **LEONOUDAKIS et al.** Protein trafficking and anchoring complexes revealed by proteomic analysis of inward rectifier potassium channel (Kir2.x)-associated proteins. *J Biol Chem,* 2004, vol. 279 (21), 22331-46
- **GONG et al.** Imaging neural spiking in brain tissue using FRET-opsin protein voltage sensors. *Nat Commun,* 2014, vol. 5, 3674
- **ZUBER et al.** A membrane-targeting signal in the amino terminus of the neuronal protein GAP-43. *Nature,* 1989, vol. 341 (6240), 345-8
- **RHEE et al.** In vivo imaging and differential localization of lipid-modified GFP-variant fusions in embryonic stem cells and mice. *Genesis,* 2006, vol. 44 (4), 202-18
- **HARRISON et al.** A convenient method for the construction and expression of GPI-anchored proteins. *Nucleic Acids Res,* 1994, vol. 22 (18), 3813-4
- **NGUYEN et al.** Engineering prokaryotic channels for control of mammalian tissue excitability. *Nat Commun.,* 2016, vol. 7, 13132
- **JACKMAN et al.** Engineered cardiac tissue patch maintains structural and electrical properties after epicardial implantation. *Biomaterials,* 2018, vol. 159, 48-58
- **SHADRIN et al.** Cardiopatch platform enables maturation and scale-up of human pluripotent stem cell-derived engineered heart tissues. *Nat Commun.,* 2017, vol. 8, 1825
- **WANG et al.** Fibroblast growth factor homologous factor 13 regulates Na+ channels and conduction velocity in murine hearts. *Circ Res.,* 2011, vol. 109, 775-82
- **ZHANG et al.** STIM1-Ca2+ signaling modulates automaticity of the mouse sinoatrial node. *Proc Natl Acad Sci USA,* 2015, vol. 112 (41), E5618-27
- **LI et al.** Isolation and culture of adult mouse cardiomyocytes for cell signaling and in vitro cardiac hypertrophy. *J Vis Exp,* 2014, vol. 87